# EUROPÄISCHE PATENTSCHRIFT

(11) **EP 4 094 748 B1**
(45) Veröffentlichungstag und Bekanntmachung des Hinweises auf die Patenterteilung: **20.09.2023**
(21) Anmeldenummer: 21315089.9
(22) Anmeldetag: 26.05.2021
(51) Int. Cl.: A61K 6/62, A61K 6/64, A61K 6/887

(54) **DENTALWERKSTOFFE AUF BASIS VON REDOXSYSTEMEN MIT OLIGOMEREN CUMOLHYDROPEROXID-DERIVATEN**
DENTAL MATERIALS BASED ON REDOX SYSTEMS WITH OLIGOMERIC CUMOLHYDROPEROXIDE DERIVATIVES
MATIÈRES DENTAIRES À BASE DE SYSTÈME REDOX AVEC DÉRIVÉS DE HYDROPEROXYDE DE CUMÈNE OLIGOMÈRIQUES

(43) Veröffentlichungstag der Anmeldung: 30.11.2022
(73) Patentinhaber: Ivoclar Vivadent AG, 9494 Schaan (LI); Université Montpellier, 34090 Montpellier (FR); Centre national de la recherche scientifique, 75016 Paris (FR); Ecole Nationale Superieure de Chimie de Montpellier - ENSCM, 34090 Montpellier (FR)
(72) Erfinder: MOSZNER, Norbert, 9495 Triesen (LI); CATEL, Yohann, 9497 Buch (CH); ANGERMANN, Jörg, 7320 Sargans (CH); ROBIN, Jean-Jacques, 34830 Clapiers (FR); MORANDI, Paul, 86510 Chaunay (FR); MONGE-DARCOS, Sophie, 34380 Viols Le Fort (FR)
(74) Vertreter: Uexküll & Stolberg

(56) Entgegenhaltungen:
- EP-A1- 0 328 643
- EP-A1- 3 692 976
- EP-A2- 0 250 090

## Beschreibung

Die vorliegende Erfindung betrifft radikalisch polymerisierbare Zusammensetzungen mit einem Cumolhydroperoxid-Redoxinitiatorsystem, das oligomere Cumolhydroperoxid-Derivate enthält. Die Zusammensetzungen eignen sich besonders als Dentalmaterialien, beispielsweise als Prothesenmaterialien, Zemente, Adhäsive und Komposite für direkte Füllungen.

Die Haupteinsatzgebiete von Polymeren im Dentalbereich sind die abnehmbare (z.B. Zähne und Prothesenbasismaterialien) und festsitzende Prothetik (z.B. Verblendmaterialen, Kronen oder Zemente), Füllungsmaterialien (z.B. direkte oder indirekte Füllungskomposite, Befestigungszemente oder Adhäsive) sowie Hilfsmaterialien (z.B. Abformmaterialien). Die Polymere werden gewöhnlich durch radikalische Polymerisation geeigneter Zusammensetzungen erhalten, die eine polymerisierbare organische Matrix enthalten, in der Regel eine Mischung von Monomeren, Initiator-Komponenten und Stabilisatoren.

Als Monomere werden meistens Methylmethacrylat (MMA) (Prothesenmaterialien), Mischungen von funktionalisierten Monomeren, wie z.B. 2-Hydroxyethylmethacrylat (HEMA), oder säuregruppenhaltigen Haftmonomeren, wie z.B. 10-Methacryloyloxydecyldihydrogenphosphat (MDP), mit Dimethacrylaten (Adhäsive) oder Mischungen eingesetzt, die ausschließlich Dimethacrylate enthalten (Kompositzemente und Füllungskomposite). Häufig verwendete Dimethacrylate sind 2,2-Bis[4-(2-hydroxy-3-methacryloyloxypropyl)phenyl]propan (Bis-GMA) und 1,6-Bis-[2-methacryloyloxy-ethoxycarbonylamino]-2,2,4-trimethylhexan (UDMA), die eine hohe Viskosität haben und zu Polymerisaten mit sehr guten mechanischen Eigenschaften führen. Als reaktive Verdünner finden vor allem Triethylenglycoldimethacrylat (TEGDMA), 1,10-Decandioldimethacrylat (D₃MA) oder Bis(3-methacryloyloxymethyl)tricyclo-[5.2.1.02.6]-decan (DCP) Anwendung.

Die Aushärtung von methacrylatbasierenden Dentalmaterialien erfolgt durch radikalische Polymerisation, wobei je nach Anwendungsgebiet radikalische Photoinitiatoren (Lichthärtung, direkte Füllungskomposite und Adhäsive), thermische Initiatoren (indirekte Komposite oder Prothesenmaterialien) oder Redox-Initiatorsysteme (Kompositzemente) eingesetzt werden. Außerdem ist die Kombination von Photoinitiatoren mit Redoxinitiatoren bekannt, z.B. bei Füllungen tiefer Kavitäten.

Redoxsysteme kommen vor allem dann zum Einsatz, wenn z.B. bei Prothesenmaterialien wegen einer geringen Monomerreaktivität oder bei Befestigungszementen aufgrund unzureichender Durchstrahlung eine unvollständige Aushärtung zu befürchten ist.

Um eine ausreichende Lagerstabilität der Materialien zu gewährleisten, werden Werkstoffe auf der Basis von Redoxinitiatoren meist als sogenannte ZweiKomponenten-Systeme (2K-Systeme) eingesetzt, wobei das Oxydationsmittel (Peroxid- oder Hydroperoxid) und das Reduktionsmittel (Amine, Sulfinsäuren, Barbiturate, Thioharnstoff etc.) in zwei getrennte Komponenten des Werkstoffs eingearbeitet werden. Diese Komponenten werden kurz vor der Anwendung miteinander gemischt.

Für dentale Kompositzemente wurden für lange Zeit vor allem Redoxinitiatorsysteme eingesetzt, die auf einer Mischung von Dibenzoylperoxid (DBPO) mit tertiären aromatischen Aminen, wie z.B. N,N-Diethanol-p-toluidin (DEPT), N,N-Dimethyl-sym.-xylidin (DMSX) oder N,N-Diethyl-3,5-di-tert.-butylanilin (DABA) beruhen. Aufgrund der begrenzten thermischen Stabilität von DBPO müssen darauf basierende Materialien im Kühlschrank gelagert werden. Ein weiterer Nachteil solcher DBPO/Amin-Systeme sind Verfärbungen, die durch eine langsame Oxidation der Amine verursacht werden. Außerdem wird die Radikalbildung bei DBPO/Amin-basierenden Redoxinitiatorsystemen durch Säuren und damit auch durch saure Monomere beeinträchtigt, die regelmäßig zur Herstellung von Schmelz-Dentin-Adhäsiven eingesetzt werden. Die Aminkomponente wird durch eine Säure-Base-Reaktion protoniert und dadurch deaktiviert.

Die voranstehenden Nachteile können mit Hydroperoxid-Redoxinitiatorsystemen zum Teil überwunden werden, weil keine tertiären Amine als Reduktionsmittel erforderlich sind. Außerdem sind Hydroperoxide thermisch stabiler als Peroxide. Cumolhydroperoxid weist beispielsweise eine 10-Stunden-Halbwertstemperatur T_{1/2} von 158 °C auf, die 10-Stunden-Halbwertstemperatur T_{1/2} von DBPO beträgt nur 73 °C.

Die DE 26 35 595 C2 offenbart polymerisierbare Zahnfüllmassen, die als Initiatorsystem ein substituiertes Thioharnstoff-Reduktionsmittel in Kombination mit einem Hydroperoxid-Oxydationsmittel enthalten. Die Materialien sollen eine verbesserte Farbbeständigkeit, eine hervorragende Härtegeschwindigkeit und eine verbesserte Lagerfähigkeit aufweisen.

Die EP 1 693 046 B1 offenbart dentale Zemente und Stumpfaufbaumaterialien, die ein (2-Pyridyl)-2-thioharnstoffderivat in Kombination mit einem Hydroperoxid enthalten, in dem die Hydroperoxidgruppe an ein tertiäres Kohlenstoffatom gebunden ist.

Die WO 2007/016508 A1 offenbart eine polymerisierbare dentale Zusammensetzung, die als Initiatorsystem ein Thioharnstoffderivat in Kombination mit einem Hydroperoxid enthält. Die Zusammensetzung enthält keine Monomeren mit Säuregruppen.

Gemäß EP 1 754 465 B1 lässt sich die Reaktivität des Cumolhydroperoxid/Acetylthioharnstoff-Systems durch die Zugabe von löslichen Kupferverbindungen steigern.

Die US 7,275,932 B2 schlägt die Verwendung von Hydroperoxiden und Thioharnstoffderivaten in Kombination mit einer sauren Verbindung als Beschleuniger vor. Bevorzugte saure Verbindungen sind säuregruppenhaltige Acrylate und Methacrylate wie z.B. Methacrylsäure.

Die EP 2 233 544 A1 und die EP 2 258 336 A1 offenbaren Dentalwerkstoffe, die ein Hydroperoxid und ein Thioharnstoffderivat in Kombination mit einer Vanadiumverbindung als Beschleuniger enthalten.

Nachteilig an Cumolhydroperoxid ist sein typisch aromatischer Geruch, der an Xylole und Toluol erinnert und der vor allem bei Materialien für die intraorale Anwendung als unangenehm empfunden wird.

Die EP 3 692 976 A1 offenbart geruchsarme Cumolhydroperoxid-Derivate, die sich als Initiator für Dentalwerkstoffe eignen und die sich zudem durch eine große Lagerstabilität auszeichnen.

Die EP 0 328 643 A1 offenbart α-Hydroxyperoxyisopropylphenylverbindungen mit einer oder zwei Hydroperoxidgruppen, die sich zum Nachweis von okkultem Blut eignen sollen.

Die EP 0 250 090 A2 offenbart latent härtbare Acrylformulierungen mit polymeren Hydroperoxiden, die lagerstabil und bei Umgebungstemperatur unter anaeroben Bedingungen härtbar sein sollen. Die Zusammensetzungen sollen sich zum Verkleben von Stahl und Aluminium eignen.

Der Erfindung liegt die Aufgabe zugrunde, geruchsarme Hydroperoxide zur Verfügung zu stellen, die sich als Initiatoren für die radikalische Polymerisation eignen und die ein verringertes Risiko für toxische Nebenwirkungen aufweisen. Sie sollen insbesondere für die Herstellung von Dentalwerkstoffen tauglich sein.

Diese Aufgabe wird durch Cumolhydroperoxid (CHP)-Oligomere gemäß der folgenden Formel (I) gelöst: 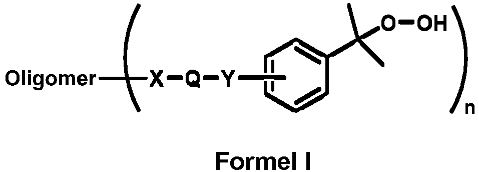 in der die Variablen die folgenden Bedeutungen haben:
- OLIGOMER: eine (Meth)acrylat- oder Styrol-basierende Copolymerkette, die n-fach durch die in Klammern stehende Gruppe substituiert ist,
- Q: ein zweiwertiger, linearer oder verzweigter aliphatischer C₁-C₁₀-Rest, der durch 1 bis 3 O-Atome unterbrochen sein kann und 1 bis 3 OH- oder OR²-Substituenten tragen kann, wobei R² ein aliphatischer, linearer oder verzweigter C₁-C₅-Alkylrest ist,
- X, Y: unabhängig voneinander entfällt, eine Ether-, Ester- oder Urethan-Gruppe, wobei die Substitution am Aromaten in 4-Stellung erfolgt, und
- n: ein Wert von 5 bis 20, und
wobei das Cumolhydroperoxidoligomer eine zahlenmittlere Molmasse von 1000 bis 10 000 g/mol hat, gemessen mittels Gel-Permeations-Chromatographie (GPC).

Alle hierin gezeigten Formeln erstrecken sich nur auf solche Verbindungen, die mit der chemischen Valenzlehre vereinbar sind. Der Hinweis, dass ein Rest z.B. durch ein oder mehrere O-Atome unterbrochen ist, ist so zu verstehen, dass diese Atome jeweils in die Kohlenstoffkette des Restes eingeschoben werden. Diese Atome sind damit beidseitig durch C-Atome begrenzt und können nicht endständig sein. C₁-Reste können nicht unterbrochen oder verzweigt sein. Ester- und Urethan-Gruppen können in beliebiger Orientierung angeordnet sein. Unter aromatischen Kohlenwasserstoffresten werden der üblichen Nomenklatur entsprechend auch solche Reste verstanden, die aromatische und nicht aromatische Gruppen enthalten.

Die für die einzelnen Variablen angegebenen bevorzugten, besonders bevorzugten und ganz besonders bevorzugten Definitionen können jeweils unabhängig voneinander ausgewählt werden. Verbindungen, in denen alle Variablen die bevorzugten, besonders bevorzugten und ganz besonders bevorzugten Definitionen aufweisen, sind naturgemäß erfindungsgemäß besonders geeignet.

Eine direkte Synthese und Polymerisation von cumolhydroperoxidgruppenhaltigen Monomeren ist wegen ihrer geringen Stabilität nur schwierig möglich. Die erfindungsgemäßen CHP-Oligomere der Formel I werden daher vorzugsweise über eine dreistufige Synthese hergestellt.

In der **1. Stufe** werden zunächst polymerisationsfähige Isopropylbenzolmonomere durch Veresterung z.B. eines COOH-funktionalisierten Isopropylbenzolderivats, vorzugsweise 4-Isopropylbenzoesäure, mit einem OH-funktionalisierten Monomer hergestellt. Dabei sind OH-funktionalisierte Monomere mit radikalisch polymerisationsfähigen Styrolgruppen und vorzugsweise (Meth)acrylatgruppen als Polymerisationsgruppe (PG), wie z.B. 2-Hydroxyethylmethacrylat (HEMA), bevorzugt:
Allgemein: 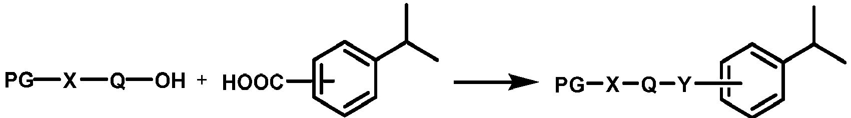
Konkretes Beispiel:

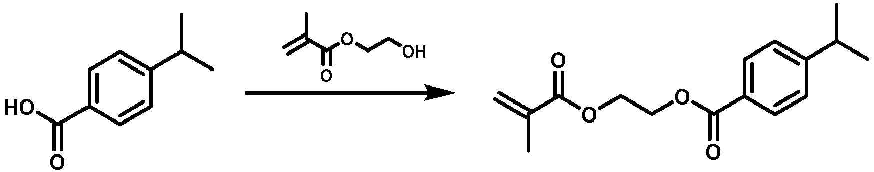

In der **2. Stufe** wird das so erhaltene polymerisationsfähige Isopropylbenzolmonomer mit den bekannten Methoden der radikalischen Polymerisation in Gegenwart eines Initiators, wie z.B. AIBN (Azobisisobutyronitril), homo- oder copolymerisiert. Bei der Homopolymerisation bildet sich ein Homopolymer mit Isopropylbenzolmonomer-Wiederholungseinheiten, das einen Polymerisationsgrad von n aufweist. Zur Molmassenkontrolle wird vorzugsweise ein Kettenregler eingesetzt, z.B. ein Mercaptan R-SH. In diesem Fall trägt die Polymerkette eine Endgruppe (EG) mit der Formel -S-R:
Allgemein Homopolymerisation: 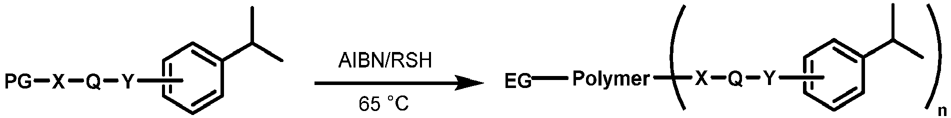
Konkretes Beispiel:

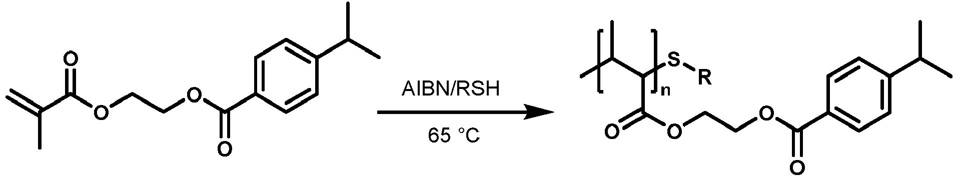

Gemäß einer bevorzugten Ausführungsform wird das polymerisationsfähige Isopropylbenzolmonomer mit einem oder mehreren radikalisch polymerisierbaren Comonomeren copolymerisiert. Das polymerisationsfähige Isopropylbenzolmonomer und die verwendeten Comonomere enthalten vorzugsweise (Meth)acrylatgruppen und bilden bei der Polymerisation somit Poly(meth)acrylatketten, die durch Isopropylbenzolgruppen und Estergruppen -COOR' substituiert sind. Die Anordnung der Monomerbausteine in der Copolymerkette kann alternierend, blockförmig oder vorzugsweise statistisch sein. Eine radikalische Copolymerisation führt meist zu einer statistischen Anordnung der Monomerbausteine. Blockcopolymere sind mit bekannten Methoden der kontrollierten radikalischen Polymerisation erhältlich. Die Zusammensetzung der gebildeten Copolymeren wird vor allem durch die Zusammensetzung der eingesetzten Comonomermischung bestimmt. Auch hier wird vorzugsweise ein Kettenregler eingesetzt, wie z.B. ein Mercaptan R-SH:
Allgemein Copolymerisation: 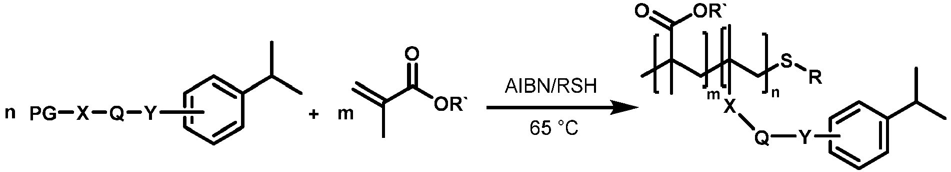
Konkretes Beispiel: Copolymerisation mit Methylmethacrylat (MMA):

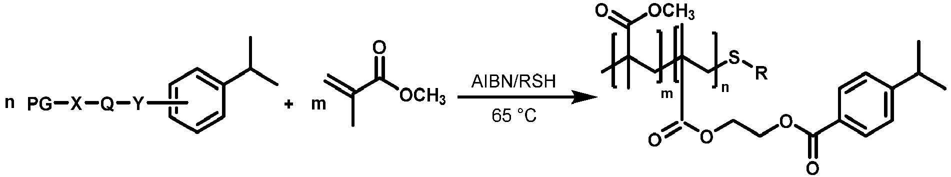

In der **3. Stufe** werden dann in einer polymeranalogen Reaktion die in der 2. Stufe erhaltenen Homo- oder Copolymeren durch Oxydation der Isopropylgruppen, z.B. mit Luftsauerstoff in Gegenwart von AIBN und N-Hydroxyphthalimid (NHPI), ohne Änderung des Polymerisationsgrades in die erfindungsgemäßen CHP-Oligomere der Formel I überführt:
Allgemein: 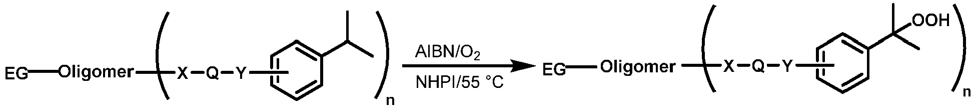
Konkretes Beispiel:

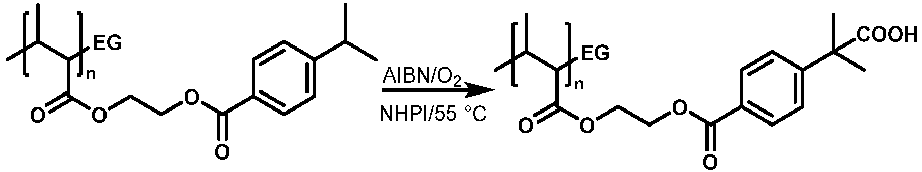

Die Oxydation der Isopropylgruppen zu Hydroperoxidgruppen verläuft nicht ganz vollständig, so dass die Oligomere noch eine geringe Menge an Isopropylgruppen enthalten können. Bevorzugt sind Oligomere, in denen mindestens 70 %, besonders bevorzugt mindestens 75 %, ganz besonders bevorzugt mindestens 80 % und am meisten bevorzugt mindestens 85 % der Isopropylgruppen zu Hydroperoxidgruppen umgesetzt wurden.

Enthält das Ausgangspolymer eine Thioether-Endgruppe, wird diese vor der Oxidation der Isopropylbenzolgruppen vorzugsweise in eine Sulfongruppe überführt, vorzugsweise durch Umsetzung mit Magnesiummonoperoxyphthalat-Hexahydrat.

Bevorzugte Comonomere zur Synthese der erfindungsgemäßen CHP-Oligomere der Formel I sind (Meth)acrylate mit der allgemeinen Formel H₂C=CR‴-COOR', in der R‴ H oder Methyl ist, und R' ein linearer oder verzweigter C₁-C₁₀-Alkylrest, der unsubstituiert oder durch eine oder mehrere funktionelle Gruppen, vorzugsweise - OH, -COOH und/oder -Cl, substituiert sein kann, Benzyl oder Furfuryl ist. Besonders bevorzugte Comonomere sind Methyl-, Ethyl-, Propyl-, Butyl-, Hexyl-, 2-Hydroxyethyl, Hydroxypropyl-, Benzyl-, Furfuryl-, Isobornyl-, 2-Ethylhexyl(meth)acrylat und Mischungen davon, wobei in allen Fällen die Methacrylate ganz besonders bevorzugt sind. Durch die Verwendung von Comonomeren kann z.B. die Löslichkeit der CHP-Oligomere der Formel I in Monomermischungen verbessert werden.

Isopropylbenzolmonomere und Comonomere werden vorzugsweise in einem Molverhältnis von 0,1 bis 0,9, besonders bevorzugt von 0,5 bis 0,9 eingesetzt. Mit zunehmendem Gehalt an Cumolhydroperoxidgruppen in den CHP-Oligomeren der Formel I verringert sich die zur Initiierung der radikalischen Polymerisation erforderliche Menge an CHP-Oligomeren in darauf basierenden radikalisch polymerisierbaren Zusammensetzungen entsprechend.

CHP-Cooligomere können außerdem erhalten werden, indem in Stufe 2 unterschiedliche Isopropylbenzolmonomere copolymerisiert werden.

Gemäß einer anderen Ausführungsform der Erfindung werden die erfindungsgemä-βen CHP-Oligomere der Formel I hergestellt, indem in der 1. Stufe Copolymere mit geeigneten reaktiven Seitengruppen, wie Hydroxyl- oder Carboxyl-Gruppen, hergestellt werden, vorzugsweise durch radikalische Copolymerisation von 2-Hydroxyethyl-(meth)acrylat oder (Meth)acrylsäure mit anderen Mono(meth)acrylaten. In der 2.Stufe werden die so erhaltenen OH- oder COOH-funktionalisierten Copolymeren dann durch polymeranaloge Reaktion mit einem entsprechenden Isopropylbenzol-Derivat verestert, z.B. mit 4-Isopropylbenzoesäure im Falle von OH-gruppenhaltigen Polymeren oder 4-Isopropylphenol im Falle von COOH-haltigen Polymeren. In der 3. Stufe werden die in der 2. Stufe hergestellten Homo- oder Copolymeren durch Oxidation der Isopropylguppen in die erfindungsgemäßen CHP-Oligomere der Formel I überführt, z.B. durch Reaktion mit Luftsauerstoff in Gegenwart von AIBN und N-Hydroxyphthalimid (NHPI).

Gemäß einer weiteren Ausführungsform der Erfindung werden in der 1.Stufe isocyanatgruppenhaltige Copolymere hergestellt, vorzugsweise durch radikalische Copolymerisation von (Meth)acrylaten mit 2-Isocyanatoethylmethacrylat, die dann in der 2. Stufe z.B. mit p-Isopropylbenzylalkohol umgesetzt werden. In der 3. Stufe werden nach Oxidation der Isopropylguppen, z.B. mit Luftsauerstoff in Gegenwart von AIBN und N-Hydroxyphthalimid (NHPI), die erfindungsgemäßen CHP-Oligomere der Formel I erhalten.

Die erfindungsgemäßen Oligomere werden zur Begrenzung der Kettenlänge vorzugsweise unter Verwendung eines Kettenüberträgers hergestellt. Kettenüberträger werden auch als Kettenregler bezeichnet. Erfindungsgemäß bevorzugte Kettenregler sind Mercaptane, insbesondere Mercaptane der Formel R-(SH)ₚ, in der R ein linearer oder verzweigter aliphatischer C₁-C₁₅-Kohlenwasserstoffrest, der durch ein oder mehrere S- und/oder O-Atome und/oder -COO- unterbrochen und der unsubstituiert oder durch eine oder mehrere Br- und/oder Cl-Atome und/oder OH- oder COOH-Gruppen substituiert sein kann, oder ein aromatischer C₆-C₁₂-Kohlenwasserstoffrest ist, und p 1 oder 2, vorzugsweise 1, ist. R ist vorzugsweise ein verzweigter und besonders bevorzugt ein linearer C₂-C₁₅-Alkylrest, der durch funktionelle Gruppen, insbesondere -Br, -Cl, -OH und/oder -COOH substituiert sein kann und der vorzugsweise nicht substituiert ist. Besonders bevorzugte Mercaptane sind Laurylmercaptan (1-Dodecanthiol, DDT), 2-Mercaptoethanol, 3-Mercaptopropanol, 3-Mercapto-2-butanol, 2-Mercapto-3-butanol, 3-Mercapto-2-methyl-butan-1-ol, 3-Mercapto-3-methyl-hexan-1-ol, 3-Mercaptohexanol, 2-Mercaptoessigsäure, 3-Mercaptopropionsäure, 4-Methylbenzenethiol, Isooctyl-3-mercaptopropionat, tert-Nonylmercaptan, 4,4'-Thiobisbenzenethiol und 1,8-Dimercapto-3,6-dioxaoctan.

Weitere bevorzugte Kettenregler sind Disulfide (R-S-S-R), insbesondere Dithiourethandisulfide, wie Tetramethylthiuramdisulfid und Isopropylxanthogensäuredisulfid. Disulfide werden auch als Photoiniferter bezeichnet, weil sie bei der radikalischen Photopolymerisation als Photoinitiator (Photoini-) wirken und gleichzeitig auch an Übertragungsreaktionen (engl.: transfer, -fer-) und Abbruchsreaktionen (engl. termination, -ter) teilnehmen.

Außerdem können Silane, insbesondere Trimethylsilan und Pentamethyldisilan, sowie halogenierte Verbindungen, insbesondere Tetrachlorkohlenstoff, Tetrabromkohlenstoff und Bromotrichlormethan, als Kettenregler eingesetzt werden.

Erfindungsgemäß besonders bevorzugte Kettelregler sind Alkylmercaptane (R-SH, wobei R ein linearer C₂-C₁₅-Alkylrest ist), Mercaptoethanol und Mercaptoessigsäure.

Der Polymerisationsgrad der erfindungsgemäßen CHP-Oligomere ergibt sich aus der Synthese. Die Zusammensetzung von Co-Oligomeren kann ¹H-NMR-spektroskopisch bestimmt werden, und der Peroxid-Gehalt n kann ¹H-NMR-spektroskopisch oder durch Titration ermittelt werden.

Die erfindungsgemäßen CHP-Oligomere der Formel I haben eine zahlenmittlere Molmasse von 1 000 bis 10 000 g/mol, besonders bevorzugt von 1 000 bis 6 000 g/mol und ganz besonders bevorzugt von 1 000 bis 3000 g/mol. Eine geringere Molmasse führt zu einer geringen Lösungsviskosität, was beim Einsatz in Harzen, Zementen oder Kompositen vorteilhaft ist.

Wenn nicht anders angegeben, handelt es sich hierin bei der Molmasse von Oligomeren und Polymeren um die zahlenmittlere Molmasse, die mittels Gel-Permeations-Chromatographie (GPC) bestimmt wird. Dabei handelt es sich um eine Relativmethode bei der die Moleküle aufgrund ihrer Größe, genauer gesagt aufgrund ihres hydrodynamischen Volumens, getrennt werden. Die Bestimmung der absoluten Molmasse erfolgt durch Kalibrierung mit bekannten Standards.

Erfindungsgemäß bevorzugte CHP-Oligomere lassen sich durch die folgende Formel II darstellen, 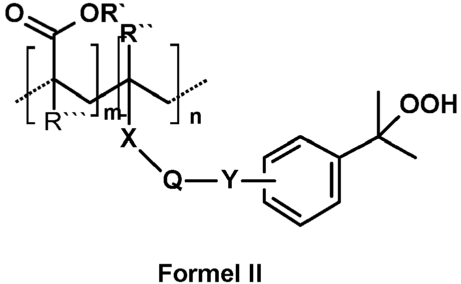 in der
- R': ein linearer oder verzweigter C₁-C₁₀-Alkylrest, der durch eine oder mehrere funktionelle Gruppen, insbesondere -OH, -COOH und/oder -Cl, substituiert sein kann oder vorzugsweise unsubstituiert ist, oder ein cyclischer C₄-C₁₀-Alkylrest, ein heterocyclischer oder isocyclischer aromatischer C₄-C₆-Kohlenwasserstoffrest ist,
- R", R‴: unabhängig voneinander jeweils H oder Methyl sind,
- m: eine Zahl von 0 bis 30, vorzugsweise 1 bis 20, ist,
- n: eine Zahl von 5 bis 20 ist und
die übrigen Variablen die oben genannten Bedeutungen haben. In Homopolymeren ist m = 0 und in Copolymeren ist m ≥ 1. Das Verhältnis von n zu m liegt vorzugsweise in einem Bereich von 0,1 bis 0,9, besonders bevorzugt 0,5 zu 0,9. Die Summe von n+m ist vorzugsweise größer als 10 und kleiner als 100.

Es entspricht dem allgemeinen Fachwissen, dass die Kettenlänge der Polymerketten variiert. Bei n und m handelt es sich somit in allen Fällen um Mittelwerte (Zahlenmittel).

In den Formeln I und II sind den üblichen Gepflogenheiten entsprechend die Endgruppen der Polymerkette nicht angegeben. Die Kettenübertragung läuft nach folgendem Schema ab:

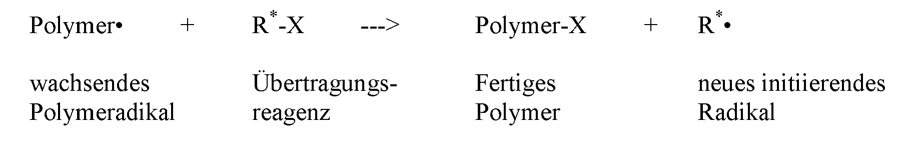

Bei der Verwendung von Kettenreglern werden demnach Ketten mit den Endgruppen R* und X erhalten. Erfindungsgemäß bevorzugte Kettenregler sind Mercaptane R-SH (R^{*} = R-S-; X = H), so dass Polymerketten erhalten werden, die ein H-Atom bzw. eine R-S-Gruppe als Endgruppen tragen, wobei die R-S-Gruppen vorzugsweise zu Sulfoxid- (-SO-R) und insbesondere Sulfongruppen (-SO₂R) oxydiert werden. Mercaptane mit zwei Mercaptogruppen R-(SH)₂ ergeben dementsprechend Endgruppen mit der Formel HS-R-S-.

Disulfide führen auf analoger Weise zu Endgruppen mit der Formel R-S-. Tetrachlorkohlenstoff (CCl₄), Tetrabromkohlenstoff (CBr₄) und Bromotrichlormethan (CBrCl₃) führen zu den folgenden Endgruppen: -CCl₃, -CBr₃ und -CCl₃.

Wenn zur Herstellung der erfindungsgemäßen Oligomere keine Kettelregler eingesetzt werden, wird die Endgruppe der Oligomere durch den zur Polymerisation verwendeten Initiator bestimmt. Erfindungsgemäß bevorzugte Initiatoren sind Azobis-(isobutyronitril) (AIBN), Diperoxide, insbesondere Dibenzoylperoxid und Di-tert-Butylperoxid, und Peroxodisulfate, insbesondere Kaliumperoxodisulfat K₂S₂O₈, Natriumperoxodisulfat Na₂S₂O₈. Diese Initiatoren führen zu den folgenden Endgruppen: Dibenzoylperoxid: C₆H₅-(CO)-O-; Di-tert-Butylperoxid: C₄H₉-O-; K₂S₂O₈: KSO₄, Na₂S₂O₈: NaSO₄. Es entspricht dem allgemeinen Fachwissen, dass außerdem durch Kombination und Disproportionierung von Polymerketten Endgruppen gebildet werden.

Bevorzugte CHP-Oligomere, die unter Verwendung von Mercaptanen als Kettenregler erhalten werden, lassen sich durch die folgende Formel IIa darstellen, 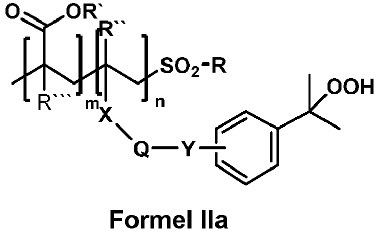 in der
- R: ein verzweigter oder vorzugsweise linearer C₂-C₁₅-Alkylrest ist, der durch eine oder mehrere funktionelle Gruppen, insbesondere -Br, -Cl, -OH und/oder -COOH, substituiert sein kann oder vorzugsweise unsubstituiert ist,
- R': ein linearer oder verzweigter C₁-C₁₀-Alkylrest, der durch eine oder mehrere funktionelle Gruppen, insbesondere -OH, -COOH und/oder -Cl substituiert sein kann oder vorzugsweise unsubstituiert ist, oder ein cyclischer C₄-C₁₀-Alkylrest, ein heterocyclischer oder isocyclischer aromatischer C₄-C₆-Kohlenwasserstoffrest ist,
- R", R‴: unabhängig voneinander jeweils H oder Methyl sind,
- m: eine Zahl von 0 bis 30, vorzugsweise 1 bis 20, ist,
- n: eine Zahl von 5 bis 20 ist und
die übrigen Variablen die oben genannten Bedeutungen haben.

In den Formeln II und IIa weist die Gruppe 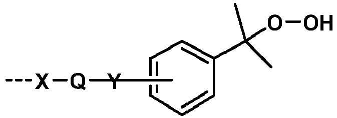 vorzugsweise eine der folgenden Strukturen auf:

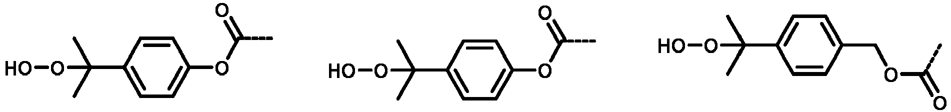

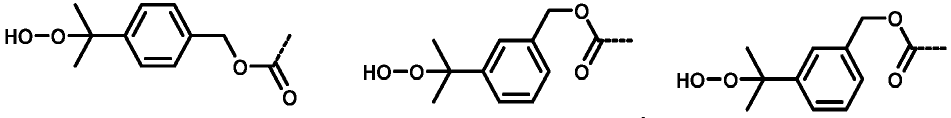

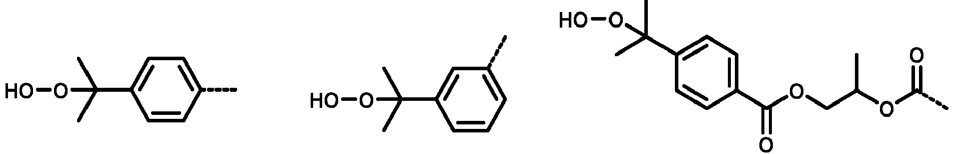

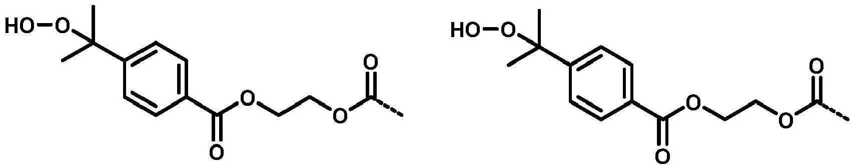

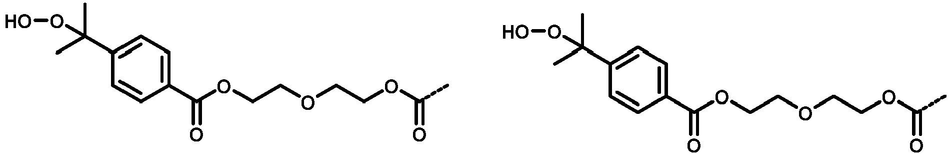

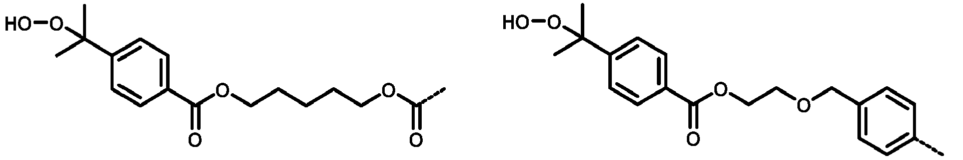

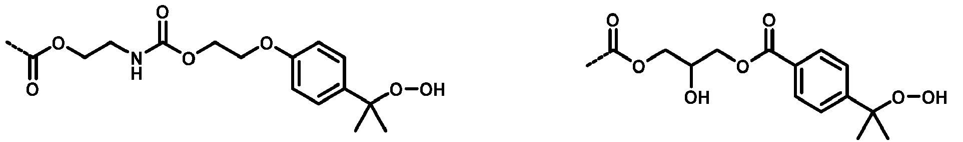

Die CHP-Oligomere der Formel I haben eine große Lagerstabilität bei Raumtemperatur, sind geruchsfrei, besitzen eine geringe Löslichkeit in Wasser und lassen sich als Hydroperoxid-Komponente in Redoxinitiatorsystemen für radikalisch polymerisierbare Zusammensetzungen einsetzen, insbesondere in Redoxinitiatorsystemen für dentale Zusammensetzungen. Ein besonderer Vorteil der erfindungsgemäßen oligo- oder polymeren Verbindungen der Formel I ist, dass sie nach der Polymerisation nicht aus den gehärteten Materialien freigesetzt werden, was im Hinblick auf mögliche toxische Nebenwirkungen für dentale und andere medizinische Anwendungen ein erheblicher Vorteil ist. Radikalisch polymerisierbare Zusammensetzungen, die mindestens ein CHP-Oligomer der Formel I enthalten, sind ebenfalls Gegenstand der Erfindung.

Die erfindungsgemäßen Zusammensetzungen enthalten neben dem CHP-Oligomer der Formel (I) vorzugsweise mindestens ein Thioharnstoffderivat als Beschleuniger. Erfindungsgemäß bevorzugte Thioharnstoffderivate sind die in der EP 1 754 465 A1 in Absatz [0009] aufgeführten Verbindungen. Besonders bevorzugte Thioharnstoffderivate sind Acetyl-, Allyl-, Pyridyl- und Phenylthioharnstoff, Hexanoylthioharnstoff und Mischungen davon. Ganz besonders bevorzugt ist Acetylthioharnstoff (ATU).

Weiter bevorzugt sind Thioharnstoffderivate mit der Formel III 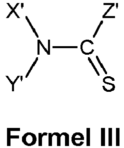 in der
- X': H oder Y' ist,
- Y': ein Alkylrest mit 1 bis 8 Kohlenstoffatomen, ein Cycloalkylrest mit 5 oder 6 Kohlenstoffatomen, ein Chlor-, Hydroxy- oder Mercapto-substituierter Alkylrest mit 1 bis 8 Kohlenstoffatomen, ein Alkenylrest mit 3 bis 4 Kohlenstoffatomen, ein Arylrest mit 6 bis 8 Kohlenstoffatomen, ein Chlor-, Hydroxy-, Methoxy- oder Sulfonyl-substituierter Phenylrest, ein Acylrest mit 2 bis 8 Kohlenstoffatomen, ein Chlor- oder Methoxy-substituierter Acylrest, ein Aralkylrest mit 7 bis 8 Kohlenstoffatomen oder ein Chlor- oder Methoxy-substituierter Aralkylrest ist, und
- Z': NH₂, NHX' oder NX'₂ ist.

Weiter bevorzugt sind außerdem cyclische Thioharnstoffderivate. Unter cyclischen Thioharnstoffderivaten werden hier solche Verbindungen verstanden, bei denen die Stickstoffatome der Thioharnstoffgruppe zusammen mit dem dazwischen liegenden Kohlenstoffatom und weiteren Kohlenstoffatomen ein heterocyclisches Ringsystem bilden. Bevorzugt sind cyclische Thioharnstoffderivate der Formel (IV): 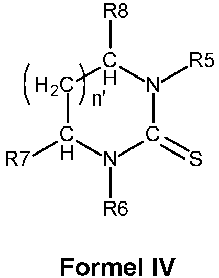 in der:
- R⁵,: R⁶ jeweils H oder ein C₁-C₄-Alkylrest sind, wobei mindestens einer dieser Reste H ist;
- R⁷, R⁸: unabhängig voneinander jeweils H, ein C₁-C₄-Alkylrest oder ein C₁-C₄Alkoxyrest sind oder zusammen mit den Kohlenstoffatomen, an die sie gebunden sind, und dem dazwischen liegenden Kohlenstoffatom einen sechsgliedrigen, carbocyclischen, aliphatischen oder aromatischen Ring bilden, der durch einen oder mehrere, vorzugsweise 1 oder 2, C₁-C₄-Alkylreste und/oder C₁-C₄-Alkoxyreste substituiert sein kann;
- n': 0, 1, 2 oder 3, vorzugsweise 0 oder 1 ist.

Vorzugsweise haben die Variablen der Formel IV die folgenden Bedeutungen:
- R⁵,: R⁶ jeweils H oder ein C₁-C₂-Alkylrest, vorzugsweise H oder Methyl, wobei mindestens einer dieser Reste H ist;
- R⁷,: R⁸ jeweils H, ein C₁-C₂-Alkylrest, vorzugsweise Methyl, ein C₁-C₂-Alkoxyrest, vorzugsweise Methoxy, oder bilden zusammen mit den Kohlenstoffatomen, an die sie gebunden sind, und dem dazwischen liegenden Kohlenstoffatom einen Benzolring, der durch einen C₁-C₂-Alkylrest, vorzugweise Methyl, oder einen C₁-C₂-Alkoxyrest, vorzugsweise Methoxy, substituiert sein kann;
- n': 0 oder 1.

Besonders bevorzugt haben die Variablen der Formel IV die folgenden Bedeutungen:
- R⁵,: R⁶ jeweils H oder Methyl, wobei mindestens einer dieser Reste H ist;
- R⁷,: R⁸ jeweils H, ein C₁-C₂-Alkylrest, vorzugsweise Methyl, oder ein C₁-C₂-Alkoxyrest, vorzugsweise Methoxy, und
- n': 1, oder
- R⁵,: R⁶ jeweils H oder Methyl, wobei mindestens einer dieser Reste H ist;
- R⁷, R⁸: bilden zusammen mit den Kohlenstoffatomen, an die sie gebunden sind, und dem dazwischen liegenden Kohlenstoffatom einen Benzolring, der durch einen C₁-C₂-Alkylrest, vorzugweise Methyl, oder einen C₁-C₂-Alkoxyrest, vorzugsweise Methoxy, substituiert sein kann und
- n': 0.

In allen Fällen umfasst die Formel IV auch die entsprechenden Isothioharnstoffderivate.

Besonders bevorzugt sind 3,4,5,6-Tehtrahydro-2-pyrimidinthiol (1), 2-Imidazolidinthion (2), 2-Mercaptobenzimidazol (4), 1-Methyl-1H-benzimidazol-2-thiol, 2-Mercapto-1-methylimidazol und 2-Mercapto-5-methoxybenzimidazol.

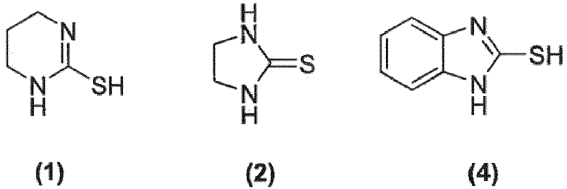

Gemäß einer ganz besonders bevorzugten Ausführungsform der Erfindung wird als Beschleuniger eine Kombination mindestens eines cyclischen Thioharnstoffderivats und mindestens eines acyclischen Thioharnstoffderivats eingesetzt.

Gemäß einer weiteren bevorzugten Ausführungsform enthalten die erfindungsgemä-βen Zusammensetzungen neben mindestens einem CHP-Oligomer der Formel I und mindestens einem Thioharnstoffderivat zusätzlich mindestens eine Übergangsmetallverbindung. Es wurde gefunden, dass die Zugabe einer Übergangsmetallverbindung Werkstoffe ergibt, die nach der Härtung erheblich verbesserte mechanische Eigenschaften aufweisen.

Erfindungsgemäß bevorzugte Übergangsmetallverbindungen sind Verbindungen, die sich von solchen Übergangsmetallen ableiten, die mindestens zwei stabile Oxidationsstufen haben. Besonders bevorzugt sind Verbindungen der Elemente Kupfer, Eisen, Kobalt, Nickel und Mangan. Diese Metalle weisen die folgenden stabilen Oxydationsstufen auf: Cu(I)/Cu(II), Fe(II)/Fe(III), Co(II)/Co(III), Ni(II)/Ni(III), Mn(II)/Mn(III). Zusammensetzungen, die mindestens eine Kupfer-Verbindung enthalten, sind besonders bevorzugt.

Die Übergangsmetalle werden bevorzugt in Form ihrer Salze eingesetzt. Bevorzugte Salze sind die Nitrate, Acetate, 2-Ethyl-hexanoate und Halogenide, wobei Chloride besonders bevorzugt sind.

Die Übergangsmetalle können weiterhin vorteilhaft in komplexierter Form eingesetzt werden, wobei Komplexe mit chelatbildenden Liganden besonders bevorzugt sind. Bevorzugte einfache Liganden zur Komplexierung der Übergangsmetalle sind 2-Ethylhexanoat und THF. Bevorzugte chelatbildende Liganden sind 2-(2-Aminoethyl-amino)ethanol, aliphatische Amine, besonders bevorzugt 1,1,4,7,10,10-Hexamethyltriethylentetramin (HMTETA), N,N,N',N",N"-Pentamethyldiethylentriamin (PMDETA), Tris[2-(dimethylamino)ethyl]amin (Me₆TREN), N,N,N',N'-Tetramethylethylendiamin (TMEDA), 1,4,8,11-Tetraaza-1,4,8,11-tetramethylcyclotetradecan (Me4CYCLAM), Diethylentriamin (DETA), Triethylentetramin (TETA) und 1,4,8,11-Tetraazacyclotetradecan (CYCLAM)); pyridinhaltige Liganden, besonders bevorzugt N,N,N',N'-Tetrakis-(2-pyridylmethyl)ethylendiamin (TPEN), N,N-Bis(2-pyridylmethyl)amin (BPMA), N,N-Bis(2-pyridylmethyl)octylamin (BPMOA), 2,2'-Bipyridin und 8-Hydroxychinolin. Ganz besonders bevorzugte Liganden sind Acetylaceton, Dimethylglyoxim, 8-Hydroxychinolin, 2,2'-Bipyridin und 1,10-Phenanthrolin.

Bei elektrisch neutralen Liganden muss die Ladung der Übergangsmetallionen durch geeignete Gegenionen ausgeglichen werden. Hierzu kommen insbesondere die oben genannten Ionen in Betracht, die zur Bildung von Salzen verwendet werden, wobei Acetate und Chloride besonders bevorzugt sind. Chloride und Komplexe zeichnen sich durch eine relativ gute Löslichkeit in Monomeren aus, die zur Herstellung von Dentalwerkstoffen eingesetzt werden.

Anstelle der Übergangsmetallkomplexe können nichtkomplexe Salze der Übergangsmetalle in Kombination mit komplexbildenden organischen Verbindungen zur Herstellung erfindungsgemäßer Zusammensetzungen eingesetzt werden, vorzugsweise in Kombination mit den oben genannten chelatbildenden Verbindungen. Die organischen Liganden bilden beim Mischen mit den Übergangsmetallsalzen die katalytisch wirksamen Komplexe. Die Verwendung solcher Kombinationen von Übergangsmetallsalzen und organischen Liganden ist bevorzugt.

Bevorzugt sind Übergangsmetallverbindungen der Metalle Kupfer, Eisen, Kobalt und Nickel.

Bevorzugte Kupfersalze sind CuCI, CuBr, CuCl₂, CuBr₂, CuI₂, Cu(II)-carboxylate (z.B. der Essigsäure oder 2-Ethylhexansäure). Bevorzugte Kupferkomplexe sind Komplexe mit den Liganden Acetylaceton, Phenanthrolin (z.B. 1,10-Phenanthrolin (Phen)), der aliphatischen Aminen, wie z.B. 1,1,4,7,10,10-Hexamethyltriethylentetramin (HMTETA), N,N,N',N",N"-Pentamethyldiethylentriamin (PMDETA), Tris[2-(dimethyl-amino)ethyl]amin (Me₆TREN).

Bevorzugte Eisensalze sind FeCl₃, FeBr₂ und FeCl₂. Bevorzugte Eisenkomplexe sind Komplexe mit den Liganden Acetylaceton, Triphenylphosphin, 4,4'-Di(5-nonyl)-2,2'-bipyridin (dNbpy) oder 1,3-Diisopropyl-4,5-dimethylimidazol-2-yliden (Prilm). Ganz besonders bevorzugt sind die Komplexe Fe(acac)₂ und FeCl₂(PPh₃)₂.

Bevorzugte Nickelsalze sind NiBr₂ und NiCl₂, bevorzugte Nickelkomplexe sind Nickelacetylacetonat und NiBr₂(PPh₃)₂.

In allen Fällen sind solche Komplexe bevorzugt, in denen das jeweilige Übergangsmetall in seiner stabilsten Oxidationsstufe vorliegt. Bevorzugt sind damit Komplexe von Cu²⁺, Fe³⁺, Ni²⁺ und Co³⁺.

Erfindungsgemäß sind Kupferverbindungen, Kupferkomplexe und insbesondere Mischungen von Kupfersalzen und komplexierenden organischen Liganden besonders bevorzugt.

Ganz besonders bevorzugt sind Zusammensetzungen, die mindestens ein CHP-Oligomer der Formel I, mindestens ein Thioharnstoffderivat und mindestens eine Übergangsmetallverbindung enthalten, wobei diese Komponenten vorzugsweise jeweils aus den oben definierten bevorzugten und besonders bevorzugten Stoffen ausgewählt sind.

Das oder die CHP-Oligomere der Formel I werden vorzugsweise in einer Menge von 0,5 bis 10 Gew.-%, besonders bevorzugt 1,0 bis 8,0 Gew.-% und ganz besonders bevorzugt 1,5 bis 5,0 Gew.-% eingesetzt.

Das oder die Thioharnstoffderivate werden vorzugsweise in einer Menge von 0,01 bis 5 Gew.-%, besonders bevorzugt 0,05 bis 2,0 Gew.-% eingesetzt.

Die Übergangsmetallverbindung wird ggf. vorzugsweise in einer Menge von 0,0001 bis 1 Gew.-%, bevorzugt 0,0005 bis 0,5 Gew.-% und besonders bevorzugt 0,0007 bis 0,020 Gew.-% eingesetzt.

Wenn nicht anders angegeben beziehen sich alle Prozentangaben hierin auf die Gesamtmasse der Zusammensetzung.

Erfindungsgemäß sind Zusammensetzungen bevorzugt, die mindestens ein radikalisch polymerisierbares Monomer enthalten, besonders bevorzugt Zusammensetzungen, die als radikalisch polymerisierbares Monomer mindestens ein mono- und/oder vorzugsweise multifunktionelles (Meth)acrylat enthalten. Unter monofunktionellen (Meth)acrylaten werden Verbindungen mit einer, unter multifunktionellen (Meth)acrylaten Verbindungen mit zwei oder mehr, vorzugsweise 2 bis 4 radikalisch polymerisierbaren Gruppen verstanden. Gemäß einer ganz besonders bevorzugten Ausführungsform enthalten die erfindungsgemäßen Zusammensetzungen mindestens ein Dimethacrylat oder eine Mischung von Mono- und Dimethacrylaten. Materialien, die intraoral gehärtet werden sollen, enthalten als radikalisch polymerisierbares Monomer vorzugsweise mono- und/oder multifunktionelle Methacrylate.

Bevorzugte multifunktionelle (Meth)acrylate sind Bisphenol-A-dimethacrylat, Bis-GMA (ein Additionsprodukt aus Methacrylsäure und Bisphenol-A-diglycidylether), ethoxy- oder propoxyliertes Bisphenol-A-dimethacrylat, wie z.B. das Bisphenol-A-dimethacrylat SR-348c (Sartomer) mit 3 Ethoxygruppen oder 2,2-Bis[4-(2-methacryl-oyloxypropoxy)phenyl]propan, UDMA (ein Additionsprodukt aus 2-Hydroxyethylmethacrylat und 2,2,4-Trimethylhexamethylen-1,6-diisocyanat), V-380 (ein Additionsprodukt aus einer Mischung von 0,7 mol 2-Hydroxyethylmethacrylat und 0,3 mol 2-Hydroxypropylmethacrylat mit 1 mol α,α,α',α'-Tetramethyl-m-xylylen-diisocyanat), Di-, Tri- oder Tetraethylenglycoldimethacrylat, Trimethylolpropantrimethacrylat, Pentaerythrittetramethacrylat sowie Glycerindi- und -trimethacrylat, 1,4-Butandioldimethacrylat, 1,10-Decandioldimethacrylat (D₃MA), Bis(methacryloyloxymethyl)tri-cyclo-[5.2.1.0^{2,6}]decan (DCP), Polyethylenglycol- oder Polypropylenglycoldimethacrylate, wie z.B. Polyethylenglycol-200- oder -400-dimethacrylat (PEG-200- oder PEG-400-DMA) oder 1, 1 2-Dodecandioldimethacrylat, oder eine Mischung davon.

Bevorzugte monofuktionelle Monomere sind Benzyl- und Furfurylmethacrylat, 2-Phenoxyethylmethacrylat, 2-(o-Biphenyloxy)ethylmethacrylat, 2-Hydroxy-3-phenoxy-propyl-methacrylat, Phenethylmethacrylat, 2-[(Benzyloxycarbonyl)-amino]-ethylmethacrylat, 2-[(Benzylcarbamoyl)oxy]-ethylmethacrylat, 1-Phenoxypropan-2-yl-methacrylat und 2-(p-Cumylphenoxy)-ethylmethacrylat, Tricylodecanmethacrylat, Tricyclodecanmethylmethacrylat und/oder 2-(p-Cumylphenoxy)ethylmethacrylat.

Gemäß einer Ausführungsform enthalten die erfindungsgemäßen Zusammensetzungen neben den oben genannten Monomeren vorzugsweise zusätzlich ein oder mehrere säuregruppenhaltige radikalisch polymerisierbare Monomere (Haftmonomere). Diese verleihen den Werkstoffen selbsthaftende und/oder selbstätzende Eigenschaften. Säuregruppenhaltige Monomere eignen sich daher besonders zur Herstellung von selbsthaftenden Dentalwerkstoffen, wie z.B. von Befestigungszementen.

Bevorzugte säuregruppenhaltige Monomere sind polymerisationsfähige Carbonsäuren, Phosphonsäuren und Phosphorsäureester sowie deren Anhydride. Bevorzugte Carbonsäuren und Carbonsäureanhydride sind 4-(Meth)acryloyloxyethyltrimellitsäureanhydrid, 10-Methacryloyloxydecylmalonsäure, N-(2-Hydroxy-3-methacryloyl-oxypropyl)-N-phenylglycin, 4-Vinylbenzoesäure. Bevorzugte Phosphorsäureester sind 2-Methacryloyloxyethyl-phenyl-hydrogenphosphat, 10-Methacryloyloxydecyldihydrogenphosphat (MDP) und Dipentaerythritpentamethacryloyloxyphosphat. Bevorzugte Phosphonsäuren sind 4-Vinylbenzylphosphonsäure, 2-[4-(Dihydroxy-phosphoryl)-2-oxa-butyl]-acrylsäure und deren Amide, Ester, wie z.B. 2-[4-(Di-hydroxyphosphoryl)-2-oxa-butyl]-acrylsäure-2,4,6-trimethylphenylester.

Besonders bevorzugte säuregruppenhaltige Monomere sind 4-Vinylbenzylphosphonsäure, 2-[4-(Dihydroxyphosphoryl)-2-oxa-butyl]-acrylsäure und deren Amide, Ester, wie z.B. 2-[4-(Dihydroxyphosphoryl)-2-oxa-butyl]-acrylsäure-2,4,6-trimethylphenyl-ester, (Meth)acrylamiddihydrogenphosphate, wie z.B. 6-Methacrylamidohexyl- oder 1,3-Bis(methacrylamido)-propan-2-yl-dihydrogenphosphat, und Mischungen davon. Diese besonders bevorzugten säuregruppenhaltigen Monomere zeichnen sich durch eine hohe Hydrolysestabilität aus.

Die erfindungsgemäßen Zusammensetzungen können neben dem erfindungsgemä-βen Initiatorsystem vorteilhaft zusätzlich einen Initiator für die radikalische Photopolymerisation enthalten. Solche Zusammensetzungen sind dualhärtend, d.h. sie lassen sich sowohl chemisch als auch durch Licht härten. Bevorzugte Photoinitiatoren sind Benzophenon und Benzoin sowie deren Derivate, α-Diketone oder deren Derivate, wie 9,10-Phenanthrenchinon, 1-Phenyl-propan-1,2-dion, Diacetyl oder 4,4'-Dichlorbenzil. Besonders bevorzugt werden Campherchinon (CC) und 2,2-Di-methoxy-2-phenyl-acetophenon und ganz besonders bevorzugt α-Diketone in Kombination mit Aminen als Reduktionsmittel, wie z.B. 4-(Dimethylamino)-benzoesäureethylester (EDMAB), N,N-Dimethylaminoethylmethacrylat, N,N-Dimethyl-sym.-xylidin oder Triethanolamin verwendet.

Die erfindungsgemäßen Zusammensetzungen enthalten vorzugsweise keine Amine. Besonders bevorzugt sind daher Norrish-Typ-I-Photoinitiatoren. Norrish-Typ-I-Photoinitiatoren benötigen keine Aminkomponente. Bevorzugte Norrish-Typ-I-Photoinitiatoren sind Acyl- oder Bisacylphosphinoxide und insbesondere Monoacyltrialkylgermanium-, Diacyldialkylgermanium- und Tetraacylgermanium-Verbindungen, wie z.B. Benzoyltrimethylgerman, Dibenzoyldiethylgerman, Bis(4-methoxybenzoyl)di-ethylgerman (Ivocerin^{®}), Tetrabenzoylgerman oder Tetrakis(o-methylbenzoyl)-german.

Außerdem lassen sich auch Mischungen der verschiedenen Photoinitiatoren einsetzen, wie z.B. Bis(4-methoxybenzoyl)diethylgerman oder Tetrakis(o-methyl-benzoyl)german in Kombination mit Campherchinon und 4-Dimethylaminobenzoesäureethylester.

Die erfindungsgemäßen Zusammensetzungen können vorteilhaft außerdem einen oder mehrere organische oder anorganische Füllstoffe enthalten. Bevorzugt sind partikuläre Füllstoffe. Füllstoffhaltige Zusammensetzungen eignen sich besonders als dentale Befestigungszemente oder Füllungskomposite.

Bevorzugte anorganische Füllstoffe sind Oxide, wie SiO₂, ZrO₂ und TiO₂ oder Mischoxide aus SiO₂, ZrO₂, ZnO und/oder TiO₂, nanopartikuläre oder mikrofeine Füllstoffe, wie pyrogene Kieselsäure oder Fällungskieselsäure, Glaspulver, wie Quarz-, Glaskeramik-, Borosilikatglaspulver oder röntgenopake Glaspulver, vorzugsweise Barium- oder Strontiumaluminiumsilikatgläser, und röntgenopake Füllstoffe, wie Ytterbiumtrifluorid, Tantal(V)-oxid, Bariumsulfat oder Mischoxide von SiO₂ mit Ytterbium(III)-oxid oder Tantal(V)-oxid. Weiterhin können die erfindungsgemäßen Zusammensetzungen faserförmige Füllstoffe, Nanofasern, Whiskers oder Mischungen davon enthalten.

Bevorzugt weisen die Oxide eine Partikelgröße von 0,010 bis 15 µm, die nanopartikulären oder mikrofeinen Füllstoffe eine Partikelgröße von 10 bis 300 nm, die Glaspulver eine Partikelgröße von 0,01 bis 15 µm, vorzugweise von 0,2 bis 1,5 µm, und die röntgenopaken Füllstoffe eine Partikelgröße von 0,2 bis 5 µm auf.

Besonders bevorzugte Füllstoffe sind Mischoxide aus SiO₂ und ZrO₂, mit einer Partikelgröße von 10 bis 300 nm, vorzugsweise 5 bis 10 nm, die z.B. durch hydrolytische Co-Kondensation von Metallalkoxiden zugänglich sind, Glaspulver mit einer Partikelgröße von 0,2 bis 1,5 µm, insbesondere röntgenopake Glaspulver von z.B. Barium- oder Strontiumaluminiumsilikatgläsern, und röntgenopake Füllstoffe mit einer Partikelgröße von 0,2 bis 5 µm, insbesondere Ytterbiumtrifluorid und/oder Mischoxide von SiO₂ mit Ytterbium(III)-oxid.

Außerdem sind als Füllstoffe gemahlene Präpolymerisate oder Perlpolymerisate (Isofüller) geeignet. Diese können ausschließliche aus organischen Polymeren oder aus organischen Polymeren bestehen, die ihrerseits mit anorganischen Füllstoffen gefüllt sind. Die Art der anorganischen Füllstoffe unterliegt hierbei keinen besonderen Einschränkungen, und es können alle bekannten anorganischen, partikulären, dentalen Füllstoffe eingesetzt werden, insbesondere die oben genannten anorganischen Füllstoffe. Mit anorganischen Füllstoffen gefüllte Polymerisate werden als organisch-anorganische Kompositfüllstoffe oder Verbundfüllstoffe bezeichnet. Zur Herstellung der gemahlenen Präpolymerisate und Perlpolymere eignen sich die oben definierten Monomere und Füllstoffe. Zusammensetzungen zur Herstellung dentaler Totalprothesen enthalten als Füllstoffe vorzugsweise ausschließlich organische Füllstoffe, besonders bevorzugt gemahlene Polymerisate oder Perlpolymerisate auf Basis von Polymethylmethacrylat (PMMA), ganz besonders bevorzugt Perlpolymerisate auf der Basis von PMMA. Füllstoffe die durch Mahlen von Quarz, röntgenopaken Gläsern, Borosilikaten, Keramik oder Präpolymeren gewonnen werden, bestehen meist aus splitterförmigen Teilen.

Wenn nicht anders angegeben, handelt es sich bei allen Partikelgrößen um gewichtsmittlere Partikelgrößen, wobei die Partikelgrößenbestimmung im Bereich von 0,1 µm bis 1000 µm mittels statischer Lichtstreuung erfolgt, vorzugsweise mit einem statischen Laserstreuungs-Partikelgrößen-Analysator LA-960 (Horiba, Japan). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine 0,1 bis 0,5%ige wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320.

Partikelgrößen kleiner als 0,1 µm werden vorzugsweise mittels Dynamischer Lichtstreuung (DLS) bestimmt. Die Messung der Partikelgröße im Bereich von 5 nm bis 0,1 µm erfolgt vorzugsweise durch Dynamische Lichtstreuung (DLS) von wässrigen Partikeldispersionen, vorzugsweise mit einem Malvern Zetasizer Nano ZS (Malvern Instruments, Malvern UK) mit einem He-Ne-Laser mit einer Wellenlänge von 633 nm, bei einem Streuwinkel von 90° bei 25°C.

Partikelgrößen kleiner als 0,1 µm können auch mittels REM- oder TEM-Spektroskopie bestimmt werden. Die Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf einem 50 Å dickem Kupfergitter (Maschenweite 300) aufgebracht, das mit Kohlenstoff beschichtet ist, und im Anschluss das Lösungsmittel verdampft.

Die Lichtstreuung nimmt mit abnehmender Partikelgröße ab, jedoch haben Füllstoffe mit geringer Partikelgröße eine größere Verdickungswirkung. Die Füllstoffe werden nach ihrer Partikelgröße unterteilt in Makrofüller und Mikrofüller, wobei Füllstoffe mit einer mittleren Partikelgröße von 0,2 bis 10 µm als Makrofüller und Füllstoffe mit einer mittlere Partikelgröße von ca. 5 bis 100 nm als Mikrofüller bezeichnet werden. Makrofüller werden z.B. durch Mahlen z.B. von Quarz, röntgenopaken Gläsern, Borosilikaten oder von Keramik gewonnen und bestehen meist aus splitterförmigen Teilen. Mikrofüller wie Mischoxide können z.B. durch hydrolytische Co-Kondensation von Metallalkoxiden hergestellt werden.

Zur Verbesserung des Verbundes zwischen den Füllstoffpartikeln und der vernetzten Polymerisationsmatrix sind die Füllstoffe vorzugsweise oberflächenmodifiziert, besonders bevorzugt durch Silanisierung, ganz besonders bevorzugt durch radikalisch polymerisierbare Silane, insbesondere mit 3-Methacryloyloxypropyltrimethoxysilan. Zur Oberflächenmodifizierung von nicht-silikatischen Füllstoffen, z.B. von ZrO₂ oder TiO₂, können auch funktionalisierte saure Phosphate, wie z.B. 10-Methacryloyloxydecyldihydrogenphosphat eingesetzt werden.

Außerdem können die erfindungsgemäßen Zusammensetzungen ein oder mehrere weitere Additive enthalten, vor allem Stabilisatoren, Farbmittel, mikrobiozide Wirkstoffe, fluoridionenabgebende Additive, Treibmittel, optische Aufheller, Weichmacher und/oder UV-Absorber.

Die erfindungsgemäßen Zusammensetzungen eignen sich besonders als Dentalwerkstoffe, insbesondere als dentale Zemente, Füllungskomposite und Verblendmaterialien sowie als Materialien zur Herstellung von Prothesen, künstlichen Zähnen, Inlays, Onlays, Kronen und Brücken. Die Zusammensetzungen eignen sich primär zur intraoralen Anwendung durch den Zahnarzt zur Behandlung geschädigter Zähne, d.h. zur therapeutischen Anwendung, z.B. als dentale Zemente, Füllungskomposite und Verblendmaterialien. Sie können aber auch nicht-therapeutisch (extraoral) eingesetzt werden, beispielsweise bei der Herstellung oder Reparatur von Dentalrestaurationen, wie Prothesen, künstlichen Zähnen, Inlays, Onlays, Kronen und Brücken.

Die erfindungsgemäßen Zusammensetzungen eignen sich außerdem als Werkstoffe zur Herstellung von Formkörpern für dentale aber auch für nicht dentale Zwecke, die z.B. mittels Gießen, Pressen und insbesondere durch generative Verfahren wie den 3D Druck oder Stereolithographie hergestellt werden können.

Zur Verwendung als Dentalwerkstoff sind erfindungsgemäß Zusammensetzungen bevorzugt, die
(a) 0,5 bis 15 Gew.-%, bevorzugt 1,0 bis 12,0 Gew.-% mindestens eines CHP-Oligomers der Formel I,
(b) 0,01 bis 5 Gew.-%, bevorzugt 0,05 bis 2,0 Gew.-% mindestens eines Beschleunigers,
(c) 5 bis 95 Gew.-%, bevorzugt 10 bis 95 Gew.-% mindestens eines radikalisch polymerisierbaren Monomers,
(d) 0 bis 80 Gew.-%, bevorzugt 10 bis 80 Gew.-% Füllstoff(e) und
(e) 0,001 bis 5 Gew.-%, bevorzugt 0,01 bis 3 Gew.-% Additiv(e) enthalten.

Alle Mengenangaben hierin beziehen sich auf die Gesamtmasse der Zusammensetzung, wenn nicht anders angegeben.

Zusammensetzungen zur Verwendung als dentale Zemente oder Füllungskomposite enthalten vorzugsweise:
(a) 0,5 bis 10 Gew.-%, bevorzugt 1,0 bis 8,0 Gew.-% und besonders bevorzugt 1,5 bis 5,0 Gew.-% mindestens eines CHP-Oligomers der Formel I,
(b) 0,01 bis 5 Gew.-%, bevorzugt 0,02 bis 3,0 Gew.-% und besonders bevorzugt 0,05 bis 2,0 Gew.-% mindestens eines Beschleunigers,
(c) 5 bis 95 Gew.-%, bevorzugt 10 bis 95 Gew.-% und besonders bevorzugt 10 bis 90 Gew.-% mindestens eines radikalisch polymerisierbaren Monomers,
(d) 0 bis 80 Gew.-% Füllstoff(e) und
(e) 0,001 bis 5 Gew.-%, bevorzugt 0,01 bis 3 Gew.-% und besonders bevorzugt 0,015 bis 2 Gew.-% an Additiven.

Der Füllungsgrad richtet sich nach dem gewünschten Anwendungszweck der Zusammensetzung. Füllungskomposite haben vorzugsweise einen Füllstoffgehalt von 50 bis 80 Gew.-%, und dentale Zemente von 10 bis 70 Gew.-%.

Zusammensetzungen zur Herstellung dentaler Totalprothesen enthalten bevorzugt:
(a) 0,5 bis 15 Gew.-%, bevorzugt 1,0 bis 12,0 Gew.-% und besonders bevorzugt 2,0 bis 10,0 Gew.-% mindestens eines CHP-Oligomers der Formel I,
(b) 0,01 bis 5 Gew.-%, bevorzugt 0,02 bis 3,0 Gew.-% und besonders bevorzugt 0,05 bis 2,0 Gew.-% mindestens eines Beschleunigers,
(c) 20 bis 95 Gew.-%, bevorzugt 30 bis 95 Gew.-% und besonders bevorzugt 35 bis 95 Gew.-% mindestens eines radikalisch polymerisierbaren Monomers,
(d) 5 bis 80 Gew.-%, bevorzugt 10 bis 70 Gew.-% und besonders bevorzugt 20 bis 60 Gew.-% Perlpolymerisat(e) und
(e) 0,001 bis 5 Gew.-%, bevorzugt 0,01 bis 3 Gew.-% und besonders bevorzugt 0,015 bis 2 Gew.-% Additiv(e).
   Besonders bevorzugt sind in allen Fällen Zusammensetzungen, die zusätzlich
(f) 0,0001 bis 1 Gew.-%, bevorzugt 0,0005 bis 0,5 Gew.-%, besonders bevorzugt 0,0007 bis 0,02 Gew.-% Übergangsmetallverbindung enthalten.

Erfindungsgemäß sind Werkstoffe bevorzugt, die zwei räumlich getrennte Komponenten umfassen, die zur Anwendung miteinander gemischt werden. Die erste Komponente (Katalysatorpaste) enthält das oder die CHP-Oligomere der Formel I, und die zweite Komponente (Basenpaste) enthält den oder die Beschleuniger, vorzugsweise ein oder mehrere Thioharnstoffderivate, und ggf. die Übergangsmetallverbindung. Basenpaste und Katalysatorpaste werden vorzugsweise in einem Volumenverhältnis von 1:1 miteinander gemischt. Durch das Mischen von Basen- und Katalysatorpaste wird die Härungsreaktion initiiert. Die oben angegebenen Zusammensetzungen beziehen sich auf die gemischten Pasten.

Zur Verwendung als Dentalwerkstoffe sind solche Zusammensetzungen besonders bevorzugt, die aus den genannten Bestandteilen bestehen, wobei die einzelnen Bestandteile vorzugsweise jeweils aus den oben genannten bevorzugten und besonders bevorzugten Stoffen ausgewählt sind. In allen Fällen kann als jeweiliger Bestandteil auch eine Mischung von mehreren Stoffen eingesetzt werden, also beispielsweise eine Mischung von Monomeren.

Die Erfindung wird im Folgenden anhand von Ausführungsbeispielen näher erläutert.

### Ausführungsbeispiele

### Beispiel 1

### Synthese von 4-Isopropylbenzoesäure-(2-methacryloyloxyethyl)-ester (IPBMEE)

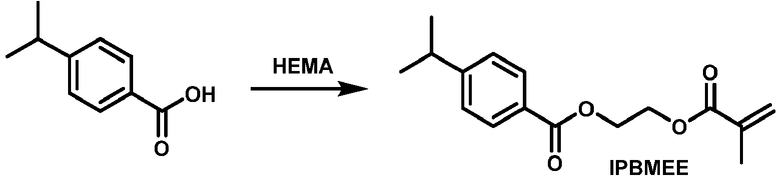

Zu einer Lösung von 52,06 g (0,40mol) 2-Hydroxyethylmethacrylat (HEMA), 59,11 g (0,36 mol) 4-Isopropyl-benzoesäure und 2,20 g (0,018 mol) 4-Dimethylaminopyridin in 250 mL Methylenchlorid wurden bei 0-5 °C 76,68 g (0,40 mol) N-(3-Dimethylaminopropyl)-N'-ethyl-carbodiimid-hydrochlorid portionsweise zugegeben.

Die gelbe Lösung wurde im auftauenden Eisbad 5 h gerührt und anschließend wässrig aufgearbeitet (3x 200 mL 1N Salzsäure, 3 x 200 mL 1N Natronlauge, 4x 200 mL deion. Wasser, 2x 100 mL gesättigte Natriumchlorid-Lösung). Die organische Phase wurde mit wasserfreiem Magnesiumsulfat getrocknet, filtriert und nach der Zugabe von 25 mg BHT unter Einleitung eines schwachen Luftstromes vom Lösungsmittel befreit. Der erhaltene Rückstand (78.9 g) wurde über Kieselgel 60 (0,03-0,2 mm) mit n-Heptan/Ethylacetat (5:1) als Eluent chromatographiert. Es wurden 49,7 g (50 % Ausbeute) **IPBMEE** als klare, farblose Flüssigkeit mit einer Reinheit von 99,66 % (HPLC) erhalten.

¹H-NMR (CDCl₃, 400 MHz): δ (ppm) = 1,26 [d, *J* = 7 Hz, 6H, HC(CH₃)₂], 1,95 (s, 3H, CH₃), 2,96 (sept, J = 7 Hz, 1H, HC(CH₃)₂], 4,47-4,50 und 4,54-4,57 (2 m, je 2H, OCH₂), 5,58 und 6,14 (2 s, je 1H, =CH₂), 7,29 und 7,97 (2 d, je J = 8 Hz, je 2H, =CH). ¹³C-NMR (CDCl₃, 100 MHz): δ (ppm) = 18,3 (CH₃), 23,7 [HC(CH₃)₂], 34,3 [HC(CH₃)₂], 66,4 und 66,5 (OCH₂), 126,0 (C=CH₂), 126,5 und 129,9 (=CH), 127,5 und 154,6 (=C), 136,0 (C=CH₂), 166,3 und 167,1 (C=O).

### Beispiel 2

### Synthese von 2-{[2-(4-Isopropylphenoxy)ethoxycarbonyl]amino}ethylmethacrylat (IPPECEM)

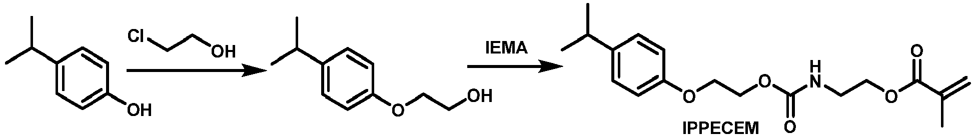

### 1. Stufe: 4-(Isopropylphenoxy)-ethanol (IPPE)

75,6 g (555 mmol) 4-Isopropylphenol wurden in Natronlauge (33,2 g (830 mmol) Natriumhydroxid in 0,5 L Wasser) aufgelöst. Nach der Zugabe von 8,85 g (27,5 mmol) Benzyltributylammoniumchlorid und 4,56 g (27,5 mmol) Kaliumiodid wurden bei 40 °C 66,8 g (830 mmol) 2-Chlorethanol zugetropft. Das trübe Reaktionsgemisch wurde 18 h bei 60 °C gerührt. Nach dem Abkühlen wurde das Gemisch mit 250 mL Toluol versetzt, für 15 min gerührt und anschließend wurden die Phasen sich trennen gelassen. Die wässrige Phase wurde noch 2x mit je 250 mL Toluol extrahiert. Die vereinigten Toluolphasen wurden nacheinander mit je 4x 100 mL 1N Natronlauge, 1N Salzsäure und Wasser gewaschen. Nach dem Trocknen der organischen Phase mit wasserfreiem Natriumsulfat wurde das Toluol im Vakuum entfernt. Die Destillation des Rohproduktes (Kp = 95-97°C_{*0*,*1 mbar*}) lieferte 84,4 g (84 % Ausbeute) 4-(Isopropyl-phenoxy)-ethanol **IPPE** als blassgelbes Öl mit einer Reinheit von 100 % (HPLC).

¹H-NMR (400 MHz, CDCl₃): δ (ppm) = 1,21 [d, *J* = 7 Hz, 6H, HC(CH₃)₂], 2,73 (t, *J* = 6 Hz, 1H, OH), 2,85 [sept, *J* = 7 Hz, HC(CH₃)₂], 3,89-3,92 (m, 2H, CH₂OH), 4,02 (t, *J* = 5 Hz, 2H, OCH₂), 6,83 und 7,12 (2 d, je *J* = 9 Hz, je 2H, =CH).

¹³C-NMR (100 MHz, CDCl₃): δ (ppm) = 24,3 [HC(CH₃)₂], 33,3 [HC(CH₃)₂], 61,5 und 69,3 (OCH₂), 114,5 (=CH_{2,6}), 127,4 (=CH_{3,5}), 141,6 (=C₄), 156,7 (=C₁).

### 2. Stufe: 2-{[2-(4-Isopropylphenoxy)-ethoxycarbonyl]-amino}-ethylmethacrylat (IPPECEM)

In 30,0 g (166 mmol) 4-(Isopropyl-phenoxy)-ethanol **IPPE** wurden 40 mg Metatin 712 (Dibutylzinndilaurat, CAS 77-58-7) sowie 16 mg BHT gelöst. Bei einer Innentemperatur von 40 °C wurde mit dem Zutropfen von 28,8 g (166 mmol) 2-Isocyanatoethylmethacrylat (IEMA) begonnen. Dabei stieg die Innentemperatur rasch an, 80 °C wurden nicht überschreiten. Nach beendeter Isocyanat-Zugabe wurde das Reaktionsgemisch für weitere 15 min bei 50 °C Badtemperatur gerührt und der Reaktionsverlauf per IR-Spektroskopie verfolgt. Nachdem keine Isocyanatreste mehr detektiert wurden, wurden 0,5 mL wasserfreies Ethanol zugegeben und nach 1 h auf Raumtemperatur abgekühlt. Das Gemisch wurde mit 100 mL Methylenchlorid verdünnt und wie folgt gewaschen: 2x mit je 50 mL 1N Natronlauge, 1x mit 50 mL 1N Salzsäure und 2x mit je 50 mL Wasser. Die organische Phase wurde mit wasserfreiem Natriumsulfat getrocknet und das Lösungsmittel im Vakuum abdestilliert. Es wurden 54,6 g (98 % Ausbeute) 2-{[2-(4-Isopropylphenoxy)-ethoxycarbonyl]-amino}-ethylmethacrylat **(IPPECEM)** als farbloses Öl mit einer Reinheit von 99,51 % (HPLC) erhalten.

¹H-NMR (400 MHz, CDCl₃): δ (ppm) = 1,21 [d, *J* = 7 Hz, 6H, HC(CH₃)₂], 1,93 (s, 3H, CH₃), 2,85 [sept, *J* = 7 Hz, HC(CH₃)₂], 3,50 (q, *J* = 5.5 Hz, 2H, NCH₂), 4,12 und 4,41 (2 q, je *J* = 5 Hz, je 2H, OCH₂CH₂O), 4,22 (q, *J* = 5 Hz, 2H, NCH₂CH₂O), 5,16 (br s, 1H NH), 5,75 und 6,11 (2 s, je 1H, =CH₂), 6,83 und 7,13 (2 d, *J* = 9 Hz, je 2H, =CH).

¹³C-NMR (100 MHz, CDCl₃): δ (ppm) = 18,3 (CH₃), 24,2 [HC(CH₃)₂], 33,3 [HC(CH₃)₂], 40,2 (NCH₂), 63,5, 63,6 und 66,4 (OCH₂), 114,4 (=CH_{2,6}), 126,0 (C=CH₂), 127,3 (=CH_{3,5}), 135,9 (C=CH₂), 141,6 (=C₄), 156,2 (=C₁, C=O*_{Urethan}*), 167,2 (C=O*_{Methacryl}*)*.*

### Beispiel 3

### Synthese eines CHP-Oligomers der Formel I: Poly[methylmethacrylat-co-{4-isopropylbenzoesäure-(2-methacryloyloxyethyl)-ester]} 1:1 (xlv = 1), mit Sulfonendgruppe (PCHP-1, Mₙ: 2600 g/mol)

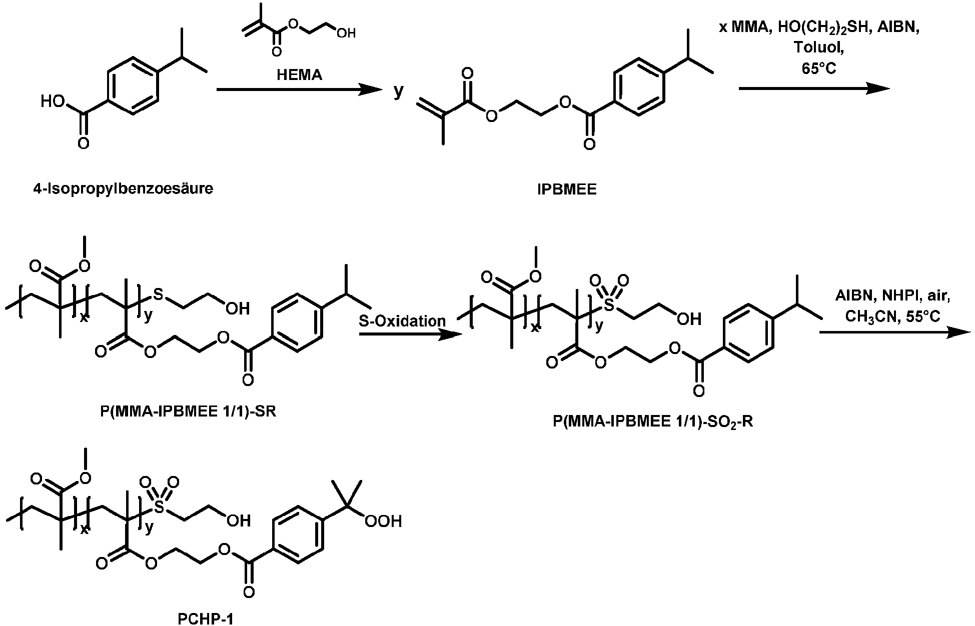

### 1. Stufe: Synthese von Poly(MMA-co-IPBMEE) 1:1 mit Thioether-Endgruppe (P(MMA-IPBMEE 1/1)-SR)

3,72 g (13,46 mmol) **IPBMEE,** 1,348 g (13,46 mmol) Methylmethacrylat (MMA), 0,044 g (0,268 mmol) Azobisisobutyronitril (AIBN), und 0,237 g (3,03 mmol) 2-Mercaptoethanol wurden in 5 mL Toluol gelöst. Danach wurde ein Stichstoffgasstrom durch die Lösung bei 0 °C geleitet und die Lösung bei 65 °C unter N₂-Atmosphäre gerührt. Nach 16 h wurde die Lösung auf Raumtemperatur abgekühlt und langsam tropfenweise unter Rühren in 500 mL Methanol bei 0°C getropft. Der ausgefallene Polymerfeststoff wurde abfiltriert und im Feinvakuum bis zur Gewichtskonstanz bei 60 °C getrocknet. Es ergaben sich 3.5 g (70 % Ausbeute) an **P(MMA- IPBMEE 1/1)-SR** als weißes Pulver.

¹H⁻NMR (CDCl₃, 400 MHz): δ (ppm) = 0,7-1,2 [CH₃CCO], 1,2-1,3 [(CH₃)₂CH], 1,5-2,1 [CH₂C], 2,4-2,7 [CH₂S], 2,9-3,0 [HC(CH₃)₂], 3,4-3,7 [OCH₃ and CH₂OH], 4,1-4,6 [CH₂OCOAr, CH₂OCOC], 7,2-8,1 [CH_{Ar}].

### 2. Stufe: Synthese von Poly(MMA-co-IPBMEE) 1:1 mit Sulfon-Endgruppe (P(MMA-IPBMEE 1/1)-SO₂-R)

3 g von **P(MMA-IPBMEE 1/1)-SR,** das 1,2 mmol Thioethergruppen enthielt, wurden in 20 mL Methylenchlorid gelöst und die Lösung auf -5 °C abgekühlt. Nach Zugabe von 10 mL Ethanol und 0,831 g (1,68 mmol) Magnesiummonoperoxiphthalat-Hexahydrat wurde die Reaktionsmischung 18 h bei Raumtemperatur gerührt. Danach wurde das Lösungsmittel im Vakuum abgezogen und der zurückgebliebene weiße Feststoff in 3 mL Toluol gelöst. Diese Lösung wurde dann langsam zu 300 mL Methanol bei 0 °C getropft. Der ausgefallene Polymerfeststoff wurde abfiltriert und im Feinvakuum bis zur Gewichtskonstanz bei 60 °C getrocknet. Es ergaben sich 2,35 g (78 % Ausbeute) an **P(MMA-IPBMEE 1/1)-SO₂-R** als weißer Feststoff.

¹H⁻NMR (CDCl₃, 400 MHz): δ (ppm) = 0,7-1,2 [CH₃CCO], 1,2-1,3 [(CH₃)₂CH], 1,5-2,1 [CH₂C], 2,9-3,0 [HC(CH₃)₂], 3,0-3,2 [CH₂SO₂], 3,4-3,7 [OCH₃], 3,9-4,1 [CH₂OH], 4,1-4,6 [CH₂OCOAr, CH₂OCOC], 7,2-8,1 [CH_{Ar}].

### 3. Stufe: Peroxidierung von P(MMA-IPBMEE 1/1)-SO₂-R) zu PCHP-1

2,35 g (enthalten 6,04 mmol Isopropylgruppen) an **P(MMA-IPBMEE 1/1)-SO₂-R,** 0,049 g (0,3 mmol) N-Hydroxyphthalimid und 0,06 g (0,365 mmol) AIBN wurden in 25 mL Acetonitril gelöst. Die Lösung wurde auf 55 °C erwärmt und ein schwacher Luftstrom durchgeleitet. Alle 24 h wurden 0,06 g (0,365 mmol) AIBN zugegeben. Nach 96 h Reaktionszeit, die Reaktion wurde mittels ¹H-NMR-Spektroskopie verfolgt, wurde die Lösung auf Raumtemperatur abgekühlt und langsam in 500 mL kaltes Wasser (4 °C) getropft. Nach Filtration der Lösung wurde der gebildete Feststoff in 25 mL Acetonitril gelöst. Diese Lösung wurde dann wieder in 500 mL kaltes Wasser getropft und damit das Polymer wieder ausgefällt. Das Umfällen wurde noch einmal wiederholt. Der farblose Feststoff wurde in einem Gefriertrockner getrocknet. Es ergaben sich 1,94 g (83 % Ausbeute) von **PCHP-1** als weißes Pulver. 88% der Isopropylgruppen waren erfolgreich in die entsprechenden Hydroperoxidgruppen umgewandelt worden. Mittels Gelpermeationschromatogaphie (GPC) wurde in Tetrahydrofuran (THF) eine zahlenmittlere Molmasse Mₙ von 2600 g/mol bestimmt, wobei n ~ 5 ist.

Die GPC-Untersuchungen wurden mittels eines GPC-Gerätes Varian 390-LC mit Brechungsindex-Detektor ausgerüstet mit 2 Säulen PLGel 5µm unter Verwendung von THF + 0,05 Gew.-% Toluol als Eluent bei 30 °C und einer Fließgeschwindigkeit von 1 mL/min durchgeführt. Lineares PMMA wurde als Standard verwendet.

¹H⁻NMR (CDCl₃, 400 MHz): δ (ppm) = 0,7-1,2 [CH₃CCO], 1,2-1,3 [(CH₃)₂CH _{Reste}], 1,5-1,7 [(CH₃)₂C], 1,5-2,1 [CH₂C], 2,9-3,0 [HC(CH₃)_{2 Reste}], 3,0-3,2 [CH₂SO₂], 3,4-3,7 [OCH₃], 3,9-4,1 [CH₂OH], 4,1-4,6 [CH₂OCOAr, CH₂OCOC], 7,4-8,1 [CH_{Ar}].

### Beispiel 4

### Synthese eines CHP-Oligomers der Formel I: Poly{methylmethacrylat-co-[4-isopropylbenzoesäure-(2-methacryloyloxyethyl)-ester]} 1:3 (x/y = 0,33), mit Sulfon-Endgruppe (PCHP-2, Mₙ: 2400 g/mol)

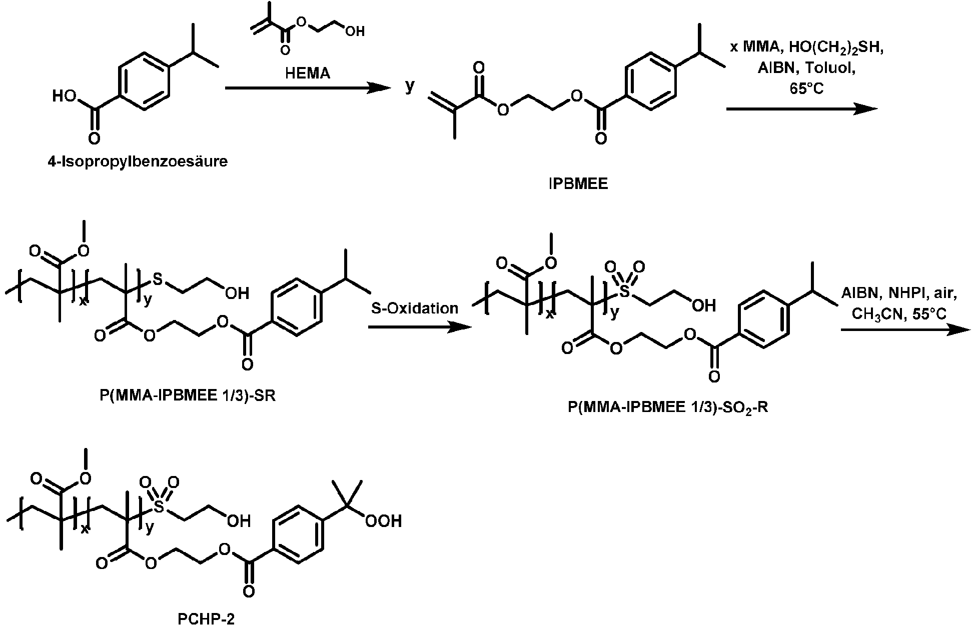

### 1.Stufe: Synthese von Poly(MMA-co-IPBMEE) 1:3 mit Thioether-Endgruppe (P(MMA-IPBMEE 1/3)-SR)

5,353 g (19,37 mmol) **IPBMEE**, 0,647 g (6,46 mmol) MMA, 0.042 g (0.254 mmol) AIBN, und 0,280 g (3,59 mmol) 2-Mercaptoethanol wurden in 6 mL Toluol gelöst. Nach Durchleiten eines N₂-Gasstroms bei 0 °C wurde die Lösung bei 65 °C unter N₂-Atmosphäre gerührt. Nach 16 h wurde die Reaktionslösung auf Raumtemperatur abgekühlt und unter Rühren in 600 mL Methanol bei 0°C getropft. Nach Filtration der Lösung wurde der gebildete Feststoff abfiltriert und im Feinvakuum bis zur Gewichtskonstanz getrocknet. Es ergaben sich 4.8 g (80 % Ausbeute) an **P(MMA-IPBMEE 1/3)-SR** als weißes Pulver.

¹H⁻NMR (CDCl₃, 400 MHz): δ (ppm) = 0.7-1.2 [CH₃CCO], 1.2-1.3 [(CH₃)₂CH], 1.5-2.1 [CH₂C], 2.4-2.7 [CH₂S], 2.9-3.0 [HC(CH₃)₂], 3.4-3.7 [OCH₃ and CH₂OH], 4.1-4.6 [CH₂OCOAr, CH₂OCOC], 7.2-8.1 [CH_{Ar}].

### 2.Stufe: Synthese von Poly(MMA-co- IPBMEE) 1:3 mit Sulfon-Endgruppe (P(MMA-IPBMEE 1/3)-SO₂-R).

3,8 g of **P(MMA-IPBMEE 1/3)-SR,** das 1,58 mmol Thioethergruppen enthielt, wurden in 25 mL Methylenchlorid gelöst. Nach Abkühlen der Lösung auf -5°C wurden 13 mL Ethanol und 1,096 g (2,22 mmol) Magnesiummonoperoxiphthalat-Hexahydrat zugegeben und die Reaktionslösung 18 h bei Raumtemperatur gerührt. Zur Aufarbeitung wurde das Lösungsmittel im Vakuum abgezogen und der weiße Feststoff in 3.8 mL Toluol gelöst. Die erhaltene Lösung wurde schließlich in 400 mL Methanol bei 0° C getropft. Der ausgefallene Polymerfeststoff wurde abfiltriert und im Feinvakuum bis zur Gewichtskonstanz bei 60 °C getrocknet. Es ergaben sich 2,94 g (77 % Ausbeute) an **P(MMA-IPBMEE 1/3)-SO₂-R** als weißer Feststoff.

¹H⁻NMR (CDCl₃, 400 MHz): δ (ppm) = 0,7-1,2 [CH₃CCO], 1,2-1,3 [(CH₃)₂CH], 1,5-2,1 [CH₂C], 2,9-3,0 [HC(CH₃)₂], 3,0-3,2 [CH₂SO₂], 3,4-3,7 [OCH₃], 3,9-4,1 [CH₂OH], 4,1-4,6 [CH₂OCOAr, CH₂OCOC], 7,2-8,1 [CH_{Ar}].

### 3. Stufe: Peroxidierung von P(MMA-IPBMEE 1/3)-SO₂-R) zu PCHP-2

2,7 g (enthalten 8,44 mmol Isopropylruppen) an **P(MMA-IPBMEE 1/3)-SO₂-R,** 0,069 g (0,42 mmol) N-Hydroxyphthalimid und 0,083 g (0,51 mmol) AIBN wurden in 27 mL Acetonitril gelöst. Die entstandene Lösung wurde auf 55 °C erwärmt und ein schwacher Luftstrom durch die Lösung geleitet. Alle 24 h wurden 0,083 g (0,51 mmol) AIBN zugegeben. Nach 96 h Reaktionszeit, die Reaktion wurde mittels ¹H-NMR-Spektroskopie verfolgt, wurde die Lösung auf Raumtemperatur abgekühlt und langsam in 500 mL kaltes Wasser (4°C) getropft. Nach Filtration der Lösung wurde der gebildete Feststoff in 27 mL Acetonitril gelöst. Diese Lösung wurde dann in 500 mL kaltes Wasser getropft und damit das Polymer wieder ausgefällt. Das Umfällen wurde noch einmal wiederholt. Der gebildete farblose Feststoff wurde in einem Gefriertrockner getrocknet. Es ergaben sich 2,59 g (96 % Ausbeute) an **PCHP-2** als weißes Pulver. 85 % der Isopropylgruppen waren erfolgreich in die entsprechenden Hydroperoxidgruppen umgewandelt worden. Mittels GPC wurde in THF eine zahlenmittlere Molmasse Mₙ von 2400 g/mol bestimmt, wobei n ~ 6 ist.

¹H⁻NMR (CDCl₃, 400 MHz): δ (ppm) = 0,7-1,2 [CH₃CCO], 1,2-1,3 [(CH₃)₂CH _{Reste}], 1,5-1,7 [(CH₃)₂C], 1,5-2,1 [CH₂C], 2,9-3,0 [HC(CH₃)_{2 Reste}], 3,0-3,2 [CH₂SO₂], 3,4-3,7 [OCH₃], 3,9-4,1 [CH₂OH], 4,1-4,6 [CH₂OCOAr, CH₂OCOC], 7,4-8,1 [CH_{Ar}].

### Beispiel 5

### Synthese eines CHP-Oligomers der Formel I: Poly{methylmethacrylat-co-[4-isopropylbenzoesäure-(2-methacryloyloxyethyl)-ester]} 1:3 (x/y = 0,33), mit Sulfon-Endgruppe (PCHP-3, Mₙ: 5000 g/mol)

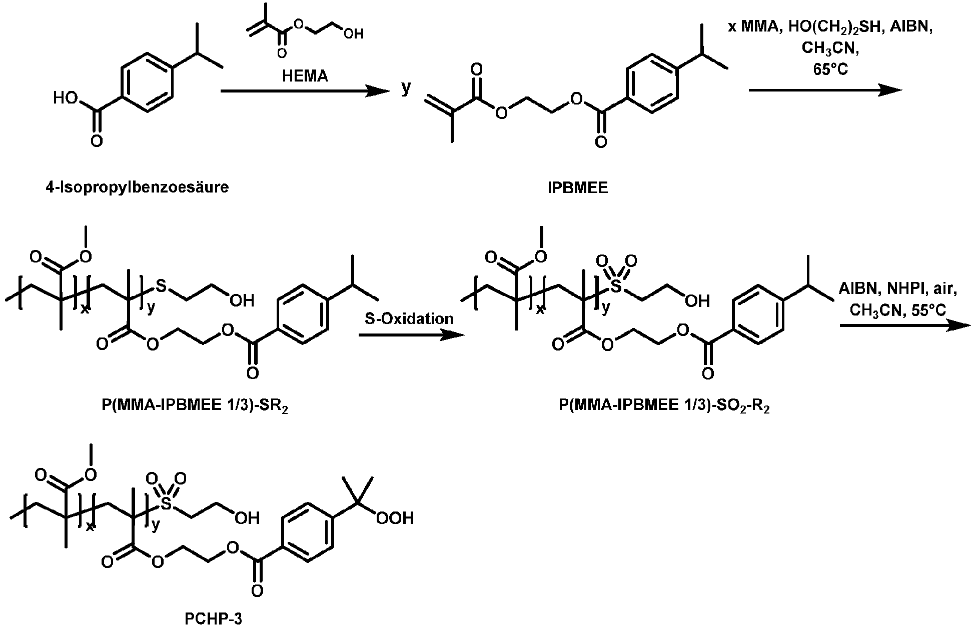

### 1. Stufe: Synthese von Poly(MMA-co-IPBMEE) 1:3 mit Thioether-Endgruppe (P(MMA-IPBMEE 1/3)-SR)

4,461 g (16,15 mmol) IPBMEE, 0,539 g (5,38 mmol) MMA, 0.035 g (0.22 mmol) AIBN, und 0,115 g (1,47 mmol) 2-Mercaptoethanol wurden in 5 mL Toluol gelöst. Nach Durchleiten eines N₂-Gasstroms bei 0 °C wurde die Lösung bei 65 °C unter N₂-Atmosphäre gerührt. Nach 16 h wurde die Reaktionslösung auf Raumtemperatur abgekühlt und unter Rühren in 500 mL Methanol bei 0°C getropft. Nach Filtration der Lösung wurde der gebildete Feststoff abfiltriert und im Feinvakuum bis zur Gewichtskonstanz getrocknet. Es ergaben sich 4,0 g (80 % Ausbeute) an **P(MMA-IPBMEE 1/3)-SR** als weißes Pulver.

¹H⁻NMR (CDCl₃, 400 MHz): δ (ppm) = 0,7-1,2 [CH₃CCO], 1,2-1,3 [(CH₃)₂CH], 1,5-2,1 [CH₂C], 2,4-2,7 [CH₂S], 2,9-3,0 [HC(CH₃)₂], 3,4-3,7 [OCH₃ and CH₂OH], 4,1-4,6 [CH₂OCOAr, CH₂OCOC], 7,2-8,1 [CH_{Ar}].

### 2. Stufe: Synthese von Poly(MMA-co- IPBMEE) 1:3 mit Sulfon-Endgruppe (P(MMA-IPBMEE 1/3)-SO₂-R)

3.0 g (enthalten 0.6 mmol Thioethergruppen) von **P(MMA-IPBMEE 1/3)-SR** wurden in 20 mL Methylenchlorid gelöst und die Lösung auf -5°C abgekühlt. Es wurden 10 mL Ethanol und 0,415 g (0,94 mmol) Magnesiummonoperoxiphthalat-Hexahydrat zugegeben und die Reaktionsmischung 18 h bei Raumtemperatur gerührt. Danach wurde das Lösungsmittel im Feinvakuum abgetrennt und der weiße Feststoff in 3,0 mL Toluol gelöst. Die Lösung wurde langsam in 300 mL Methanol bei 0°C getropft. Der ausgefallene Polymerfeststoff wurde abfiltriert und im Feinvakuum bis zur Gewichtskonstanz bei 60 °C getrocknet. Es ergaben sich 2,40 g (80 % Ausbeute) an **P(MMA-IPBMEE 1/3)-SO₂-R** als weißer Feststoff.

¹H⁻NMR (CDCl₃, 400 MHz): δ (ppm) = 0,7-1,2 [CH₃CCO], 1,2-1,3 [(CH₃)₂CH], 1,5-2,1 [CH₂C], 2,9-3,0 [HC(CH₃)₂], 3,0-3,2 [CH₂SO₂], 3,4-3,7 [OCH₃], 3,9-4,1 [CH₂OH], 4,1-4,6 [CH₂OCOAr, CH₂OCOC], 7,2-8,1 [CH_{Ar}].

### 3. Stufe: Peroxidierung von P(MMA-IPBMEE 1/3)-SO₂-R zu PCHP-3

2,0 g (enthalten 6,33 mmol Isopropylgruppen) von **P(MMA-IPBMEE 1/3)-SO₂-R,** 0,052 g (0.32 mmol) N-Hydroxyphthalimid und 0,062 g (0,38 mmol) AIBN wurden in 20 mL Acetonitril gelöst. Die entstandene Lösung wurde auf 55 °C erwärmt und ein schwacher Luftstrom durch die Lösung geleitet. Alle 24 h wurden 0,062 g (0,38 mmol) AIBN zugegeben. Nach 96 h Reaktionszeit, die Reaktion wurde mittels ¹H-NMR-Spektroskopie verfolgt, wurde die Lösung auf Raumtemperatur abgekühlt und langsam in 400 mL kaltes Wasser (4°C) getropft. Nach Filtration der Lösung wurde der gebildete Feststoff in 20 mL Acetonitril gelöst. Diese Lösung wurde dann wieder in 400 mL kaltes Wasser getropft und damit das Polymer wieder ausgefällt. Das Umfällen wurde noch einmal wiederholt. Der gebildete farblose Feststoff wurde in einem Gefriertrockner getrocknet. Es ergaben sich 1,5 g (75 % Ausbeute) an **PCHP-3** als weißes Pulver. 84 % der Isopropylgruppen waren erfolgreich in die entsprechenden Hydroperoxidgruppen umgewandelt worden. Mittels GPC wurde in THF eine zahlenmittlere Molmasse Mₙ von 4900 g/mol bestimmt, wobei n ~ 12 ist.

¹H⁻NMR (CDCl₃, 400 MHz): δ (ppm) = 0,7-1,2 [CH₃CCO], 1,2-1,3 [(CH₃)₂CH _{Reste}], 1,5-1,7 [(CH₃)₂C], 1,5-2,1 [CH₂C], 2,9-3,0 [HC(CH₃)_{2 Reste}], 3,0-3,2 [CH₂SO₂], 3,4-3,7 [OCH₃], 3,9-4,1 [CH₂OH], 4,1-4,6 [CH₂OCOAr, CH₂OCOC], 7,4-8,1 [CH_{Ar}].

### Beispiel 6

### Synthese eines CHP-Oligomers der Formel I: Poly{4-isopropylbenzoesäure-(2-methacryloyloxyethyl)-ester]} mit Sulfon-Endgruppe (PCHP-4, Mₙ: 2800g/mo/)

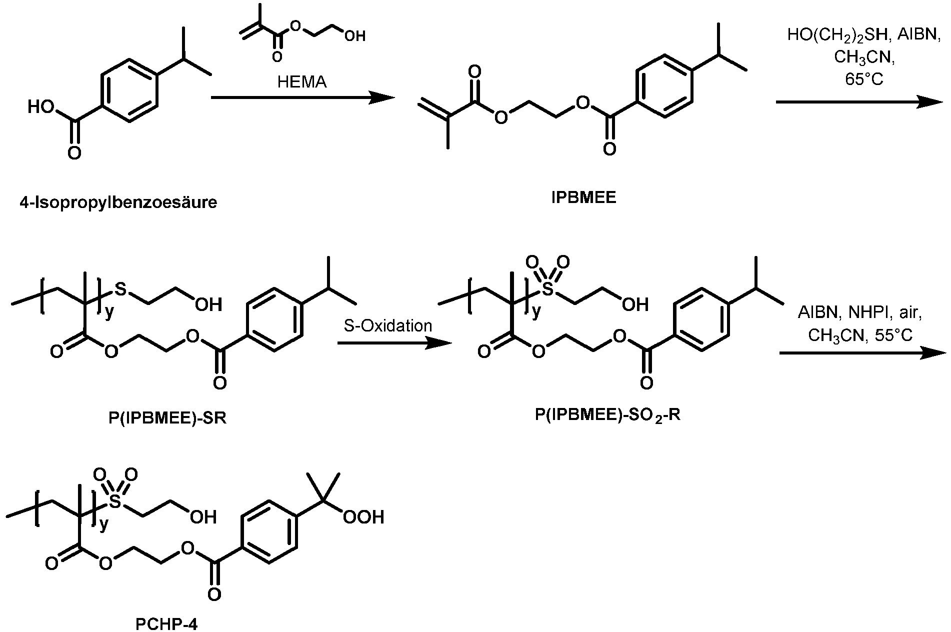

### 1. Stufe: Synthese von Poly(IPBMEE) mit Thioether-Endgruppe (PIPBMEE)-SR)

6,0 g (21,71 mmol) **IPBMEE,** 0,036 g (0,217 mmol) AIBN und 0,281 g (3,59 mmol) 2-Mercaptoethanol wurden in 6 mL Toluol gelöst. Nach Durchleiten eines N₂-Gasstroms bei 0 °C wurde die Lösung bei 65 °C unter N₂-Atmosphäre gerührt. Nach 16 h wurde die Reaktionslösung auf Raumtemperatur abgekühlt und unter Rühren in 600 mL Methanol bei 0°C getropft. Nach Filtration der Lösung wurde der gebildete Feststoff abfiltriert und im Feinvakuum bei 60 °C bis zur Gewichtskonstanz getrocknet. Es ergaben sich 3,92 g (65 % Ausbeute) an **P(IPBMEE)-SR** als weißes Pulver.

¹H⁻NMR (CDCl₃, 400 MHz): δ (ppm) = 0,7-1,2 [CH₃CCO], 1,2-1,3 [(CH₃)₂CH], 1,5-2,1 [CH₂C], 2,4-2,7 [CH₂S], 2,9-3,0 [HC(CH₃)₂], 3,5-3,6 [CH₂OH], 4,1-4,6 [CH₂OCOAr, CH₂OCOC], 7,2-8,1 [CH_{Ar}].

### 2. Stufe: Synthese von Poly(IPBMEE) Sulfon-Endgruppe (P(IPBMEE)-SO₂-R)

3,2 g (enthalten 1,143 mmol Thioethergruppen) von **P(IPBMEE)-SR** wurden in 20 mL Methylenchlorid gelöst und die Lösung auf -5°C abgekühlt. Es wurden 10 mL Ethanol und 0,791 g (1,60 mmol) Magnesiummonoperoxiphthalat-Hexahydrat zugegeben und die Reaktionsmischung 18 h bei Raumtemperatur gerührt. Danach wurde das Lösungsmittel im Feinvakuum abgetrennt und der weiße Feststoff in 3,2 mL Toluol gelöst. Die Lösung wurde langsam in 300 mL Methanol bei 0°C getropft. Der ausgefallene Polymerfeststoff wurde abfiltriert und im Feinvakuum bis zur Gewichtskonstanz bei 60 °C getrocknet. Es ergaben sich 2,74 g (86 % Ausbeute von **P(IPBMEE)-SO₂-R** als weißer Feststoff.

¹H⁻NMR (CDCl₃, 400 MHz): δ (ppm) = 0,7-1,2 [CH₃CCO], 1,2-1,3 [(CH₃)₂CH], 1,5-2,1 [CH₂C], 2,8-3,0 [HC(CH₃)₂], 3,0-3,2 [CH₂SO₂], 3,9-4,0 [CH₂OH], 4,1-4,6 [CH₂OCOAr, CH₂OCOC], 7,2-8,1 [CH_{Ar}].

### 3. Stufe: Peroxidierung von P(IPBMEE)-SO₂-R) unter Bildung von PCHP-4

2,5 g (enthalten 8,79 mmol Isopropylgruppen) von **P(IPBMEE)-SO₂-R,** 0,072 g (0,44 mmol) N-Hydroxyphthalimid und 0,087 g (0,53 mmol) AIBN wurden in 25 mL Acetonitril gelöst. Die entstandene Lösung wurde auf 55 °C erwärmt und ein schwacher Luftstrom durch die Lösung geleitet. Alle 24 h wurden 0,087 g (0,53 mmol) AIBN zugegeben. Nach 96 h Reaktionszeit, die Reaktion wurde mittels ¹H-NMR-Spektroskopie verfolgt, wurde die Lösung auf Raumtemperatur abgekühlt und langsam in 500 mL kaltes Wasser (4°C) getropft. Nach Filtration der Lösung wurde der gebildete Feststoff in 25 mL Acetonitril gelöst. Diese Lösung wurde dann wieder in 500 mL kaltes Wasser getropft und damit das Polymer wieder ausgefällt. Das Umfällen wurde noch einmal wiederholt. Der gebildete farblose Feststoff wurde in einem Gefriertrockner getrocknet. Es ergaben sich 2,25 g (90 % Ausbeute) an **PCHP-4** als weißer Pulver. 78 % der Isopropylgruppen waren erfolgreich in die entsprechenden Hydroperoxidgruppen umgewandelt worden. Mittels GPC wurde in THF eine zahlenmittlere Molmasse Mₙ von 2800 g/mol bestimmt, wobei n ~ 7 ist.

¹H⁻NMR (CDCl₃, 400 MHz): δ (ppm) = 0,7-1,2 [CH₃CCO], 1,2-1,3 [(CH₃)₂CH _{Reste}], 1,5-1,7 [(CH₃)₂C], 1,5-2,1 [CH₂C], 2,9-3,0 [HC(CH₃)_{2 Reste}], 3,0-3,1 [CH₂SO₂], 3,9-4,1 [CH₂OH], 4,1-4,6 [CH₂OCOAr, CH₂OCOC], 7,4-8,1 [CH_{Ar}].

### Beispiel 7

### Synthese eines CHP-Oligomers der Formel 1: Poly{methylmethacrylat-co-IPPECEM]} 1:1 (x/y = 1), mit Sulfon-Endgruppe (PCHP-5, Mₙ: 6000 g/mol)

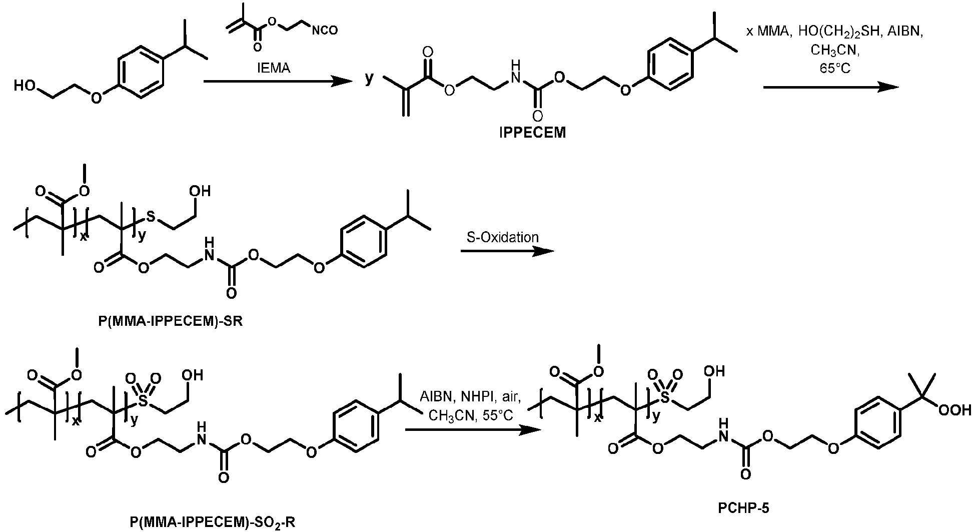

### 1. Stufe: Synthese von Poly[methylmethacrylat-co-2-{[2-(4-Isopropylphenoxy)ethoxycarbonyl]amino}ethylmethacrylat] 1:1 mit Thioether-Endgruppe (P(MMA-IPPECEM)-SR)

3,85 g (11,48 mmol) **IPPECEM,** 1,149 g (11,48 mmol) MMA, 0,038 g (0,23 mmol) AIBN und 0,115 g (1,47 mmol) 2-Mercaptoethanol wurden in 5 mL Acetonitril gelöst. Nach Durchleiten eines N₂-Gasstroms bei 0 °C wurde die Lösung bei 65 °C unter N₂-Atmosphäre gerührt. Nach 16 h wurde die Reaktionslösung auf Raumtemperatur abgekühlt und unter Rühren in 500 mL Methanol bei 0°C getropft. Nach Filtration der Lösung wurde der gebildete Feststoff abfiltriert und im Feinvakuum bei 60 °C bis zur Gewichtskonstanz getrocknet. Es ergaben sich 3,63 g (73 % Ausbeute) an **P(MMA-IPPECEM)-SR** als weißes Pulver.

¹H⁻NMR (CDCl₃, 400 MHz): δ (ppm) = 0,7-1,1 [CH₃CCO], 1,1-1,3 [(CH₃)₂CH], 1,5-2,2 [CH₂C], 2,6-2,7 [br. CH₂S], 2,8-2,9 [HC(CH₃)₂], 3,2-3,5 [CH₂NH], 3,5-3,7 [OCH₃ und CH₂OH], 3,8-4,6 [CH₂OAr, CH₂OCONH, CH₂OCOC], 5,2-5,6 [NH], 6,7-7,2 [CH_{Ar}].

### 2. Stufe: Synthese von Poly(MMA-co-IPPECEM) 1:1 mit Sulfon-Endgruppe (P(MMA-co-IPPECEM)-SO₂-R)

3 g (enthalten 0,5 mmol Thioethergruppen) von **P(MMA-IPPECEM)-SR** wurden in 20 mL Methylenchlorid gelöst und die Lösung auf -5°C abgekühlt. Es wurden 10 mL Ethanol und 0,346 g (0,70 mmol) Magnesiummonoperoxiphthalat-Hexahydrat zugegeben und die Reaktionsmischung 18 h bei Raumtemperatur gerührt. Danach wurde das Lösungsmittel im Feinvakuum abgetrennt und der weiße Feststoff in 3 mL Acetonitril gelöst. Die Lösung wurde langsam in 300 mL Methanol bei 0 °C getropft. Der ausgefallene Polymerfeststoff wurde abfiltriert und im Feinvakuum bis zur Gewichtskonstanz bei 60 °C getrocknet. Es ergaben sich 1,79 g (60 % Ausbeute) an **P(MMA-IPPECEM)-SO₂-R** als weißer Feststoff.

¹H-NMR (CDCl₃, 400 MHz): δ (ppm) = 0,7-1,1 [CH₃CCO], 1,1-1,3 [(CH₃)₂CH], 1,5-2,2 [CH₂C], 2,8-2,9 [HC(CH₃)₂], 3,0-3,2 [br. CH₂SO₂], 3,2-3,5 [CH₂NH], 3,5-3,7 [OCH₃ und CH₂OH], 3,8-4,6 [CH₂OAr, CH₂OCONH, CH₂OCOC], 5,2-5,6 [NH], 6,7-7,2 [CH_{Ar}].

### 3. Stufe: Peroxidierung von P(MMA-co-IPPECEM)-SO₂-R) zu PCHP-5

1,5 g (enthalten 3,3 mmol Isopropylgruppen) von **P(MMA-IPPECEM)-SO₂-R,** 0,027 g (0.17 mmol) N-Hydroxyphthalimid und 0,033 g (0,2 mmol) AIBN wurden in 15 mL Acetonitril gelöst. Die entstandene Lösung wurde auf 55 °C erwärmt und ein schwacher Luftstrom durch die Lösung geleitet. Alle 24 h wurden 0,087 g (0,53 mmol) AIBN zugegeben. Nach 96 h Reaktionszeit, die Reaktion wurde mittels ¹H-NMR-Spektroskopie verfolgt, wurde die Lösung auf Raumtemperatur abgekühlt und langsam in 200 mL kaltes Wasser (4°C) getropft. Nach Filtration der Lösung wurde der gebildete Feststoff in 15 mL Acetonitril gelöst. Diese Lösung wurde dann wieder in 200 mL kaltes Wasser getropft und damit das Polymer wieder ausgefällt. Das Umfällen wurde noch einmal wiederholt. Der gebildete farblose Feststoff wurde in einem Gefriertrockner getrocknet. Es ergaben sich 1,0 g (66 % Ausbeute) an **PCHP-5** als weißes Pulver. 85 % der Isopropylgruppen waren erfolgreich in die entsprechenden Hydroperoxidgruppen umgewandelt worden. Mittels GPC wurde in THF eine zahlenmittlere Molmasse Mₙ von 6000 g/mol bestimmt, wobei n ~ 11 ist.

¹H⁻NMR (CDCl₃, 400 MHz): δ (ppm) = 0,7-1,1 [CH₃CCO], 1,1-1,3 [(CH₃)₂CH_{Reste}], 1,5-2,2 [CH₃)₂C und CH₂C], 2,8-2,9 [HC(CH₃)_{2 Reste}], 3,0-3,2 [br. CH₂SO₂], 3,2-3,5 [CH₂NH], 3,5-3,7 [OCH₃ und CH₂OH], 3,8-4,6 [CH₂OAr, CH₂OCONH, CH₂OCOC], 5,2-5,6 [NH], 6,7-7,4 [CH_{Ar}].

### Beispiel 8

### Kompositzemente auf der Basis der polymeren Hydroperoxide PCHP-1, PCHP-2 und PCHP-4 aus den Beispielen 3, 4 und 6

3 Katalysator-Pasten (Kat-Pasten) A, B und C wurden aus einer Mischung der Dimethacrylate Bis-GMA (ein Additionsprodukt aus Methacrylsäure und Bisphenol-A-diglycidylether), UDMA (ein Additionsprodukt aus 2-Hydroxyethylmethacrylat und 2,2,4-Trimethylhexamethylen,1,6-diisocyanat), Triethylenglycoldimethacrylat (TEGDMA), dem Stabilisator MEHQ (Hydrochinonmonomethylether), den Initiatorkomponenten **PCHP-1, PCHP-2 oder PCHP-4** aus den Beispielen 3, 4 und 6 sowie dem Füllstoff silanisiertes Bariumaluminosilikatglas GM 27884 (0,7 µm, Schott) hergestellt (Tabelle 1).

**Tabelle 1: Zusammensetzung der Kat-Pasten A-C**

| **Komponente** | **Kat-Paste A** | **Kat-Paste B** | **Kat-Paste C** |
|---|---|---|---|
| **Bis-GMA** | 9,445 | 9,445 | 9,445 |
| **UDMA** | 12,592 | 12,592 | 12,592 |
| **TEGDMA** | 9,445 | 9,445 | 9,445 |
| **MEHQ** | 0,018 | 0,018 | 0,018 |
| **PCHP-1** | 3,500 | - | - |
| **PCHP-2** | - | 3,500 | - |
| **PCHP-4** | - | - | 3,500 |
| **GM 27884** | 65,00 | 65,00 | 65,00 |

Die Base Paste A wurde aus einer Mischung der Dimethacrylate Bis-GMA, UDMA, TEGDMA, den Stabilisatoren MEHQ (Hydrochinonmonomethylether) und TEMPO (2,2,6,6-Tetramethylpiperidinyloxyl), den Initiatorkomponenten Kupfer(II)-acetylacetonat (Cu(acac)₂) und Acetylthioharnstoff (ATU) sowie dem Füllstoff silanisiertes Bariumaluminosilikatglas GM 27884 (0,7 µm, Schott) hergestellt (Tabelle 2).

**Tabelle 2: Zusammensetzung der Base-Paste A (Angaben in Masse-%)**

| **Komponente** | **Base-Paste A** |
|---|---|
| Bis-GMA | 10,323 |
| UDMA | 13,764 |
| TEGDMA | 10,323 |
| TEMPO | 0,007 |
| MEHQ) | 0,018 |
| Cu(acac)₂ | 0,007 |
| ATU | 0,555 |
| GM 27884 | 65,00 |

Es wurden chemisch härtenden Kompositzemente bestehend aus jeweils einer Kat-Paste (**A** bis **C**) und der Base-Paste **A** hergestellt. Die Bestimmung der Biegefestigkeit und des Biege-E-Moduls erfolgte nach der Norm EN ISO-4049 (2019, Dentistry - Polymer-based filling, restorative and luting materials). Die Messung der mechanischen Eigenschaften erfolgte nach 24 h Lagerung der Prüfkörper in Wasser (WL) bei 37 °C (Tabelle 3). Die Bestimmung der Verarbeitungszeit (VZ) der Harzpasten erfolge mittels eines Motion Compact Rheometers (MCR 302 Anton Paar). Dabei wurden die Base und Kat-Pasten jeweils im Verhältnis 1:1 von Hand auf einem Mischblock vermengt. Anschließend wurde das Material am MCR-Rheometer auf ein aus Delrin bestehenden Stempel mit aufgerauter Oberfläche aufgetragen. Eine an einer Spindel befestigte Messkörperachse mit einer ebenfalls aufgerauten Oberfläche drückt die Probe zusammen und bestimmt bei leichter Rotation den Speichermodul. Beim Beginn der stabilen Phase sowie nach Erreichen einer bestimmten Steigung, wurde jeweils ein Wendepunkt definiert. Die Wendepunkte werden anschliessend durch eine Gerade verbunden. Von dieser Geraden wird der am weitesten entfernte Messpunkt als VZ definiert. Die gesamte Messung wird bei 28,7°C in einer Temperierkammer durchgeführt.

**Tabelle 3: Biegefestigkeit (BF, MPa), Biege-E-Modul (BM, MPa) und Verarbeitungszeit (VZ, s) der Zemente aus Kat-Paste A-C und Base-Paste A**

| **Komponente** | **BF 24h 37 °C H₂O (MPa)** | **BM 24h 37 °C H₂O (MPa)** | **VZ (s)** |
|---|---|---|---|
| **Kat-Paste A + Base-Paste A** | 89,5±8.2 | 6289±327 | 157±11 |
| **Kat-Paste B + Base-Paste A** | 102,2±11.2 | 6847±144 | 113±2 |
| **Kat-Paste C + Base-Paste A** | 87,8±7.8 | 6229±610 | 149±2 |

Die Ergebnisse in Tabelle 3 belegen mit Verarbeitungszeiten von 113-157 s eine gute Aushärtung, wobei die VZ im Bereich von 1-3 Min. liegen sollte. Die erhaltenen Zemente zeigen gute mechanische Eigenschaften.

## Patentansprüche

1. Cumolhydroperoxidoligomer gemäß der Formel (I): 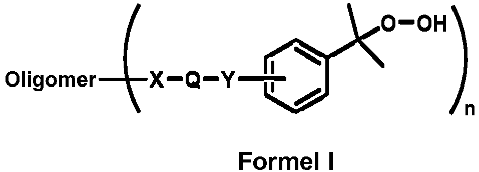
in der die Variablen die folgenden Bedeutungen haben:
OLIGOMER (Meth)acrylat- oder Styrol-basierende Copolymerkette, die n-fach durch die in Klammern stehende Gruppe substituiert ist,
Q ein zweiwertiger, linearer oder verzweigter aliphatischer C₁-C₁₀-Rest, der durch 1 bis 3 O-Atome unterbrochen sein kann und 1 bis 3 OH- oder OR²-Substituenten tragen kann, wobei R² ein ali-phatischer, linearer oder verzweigter C₁-C₅-Alkylrest ist,
X, Y unabhängig voneinander entfällt, eine Ether-, Ester- oder Urethan-Gruppe, wobei die Substitution am Aromaten in 4-Stellung erfolgt, und
n ein Wert von 5 bis 20, und
wobei das Cumolhydroperoxidoligomer eine zahlenmittlere Molmasse von 1000 bis 10 000 g/mol hat, gemessen mittels Gel-Permeations-Chromatographie (GPC).

2. Cumolhydroperoxidoligomer nach Anspruch 1, das die folgende Struktur aufweist: 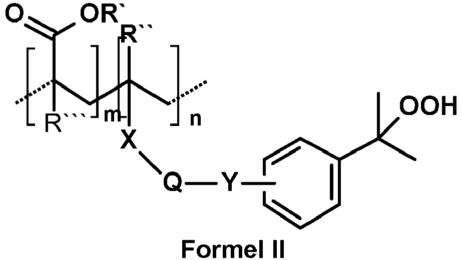 in der
R' ein linearer oder verzweigter C₁-C₁₀-Alkylrest, der durch eine oder mehrere funktionelle Gruppen, insbesondere -OH, -COOH und/oder -Cl, substituiert sein kann oder unsubstituiert ist, oder ein cyclischer C₄-C₁₀-Alkylrest, ein heterocyclischer oder isocyclischer aromatischer C₄-C₆-Kohlenwasserstoffrest ist,
R", R‴ unabhängig voneinander jeweils H oder Methyl sind,
m eine Zahl von 0 bis 30, vorzugsweise 1 bis 20, ist,
n eine Zahl von 5 bis 20 ist und
die übrigen Variablen die in Anspruch 1 genannten Bedeutungen haben.

3. Cumolhydroperoxidoligomer nach Anspruch 1, das die folgende Struktur aufweist: 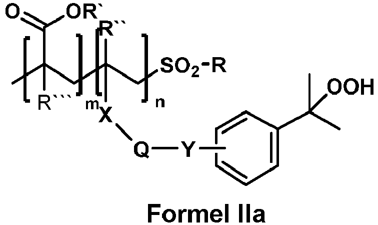 in der
R ein verzweigter oder vorzugsweise linearer C₂-C₁₅-Alkylrest ist, der durch eine oder mehrere funktionelle Gruppen, insbesondere -Br, -Cl, -OH und/oder -COOH, substituiert sein kann oder vorzugsweise unsubstituiert ist,
R' ein linearer oder verzweigter C₁-C₁₀-Alkylrest, der durch eine oder mehrere funktionelle Gruppen, insbesondere -OH, -COOH und/oder - Cl substituiert sein kann oder vorzugsweise unsubstituiert ist, oder ein cyclischer C₄-C₁₀-Alkylrest, ein heterocyclischer oder isocyclischer aromatischer C₄-C₆-Kohlenwasserstoffrest ist,
R", R‴ unabhängig voneinander jeweils H oder Methyl sind,
m eine Zahl von 0 bis 30, vorzugsweise 1 bis 20, ist,
n eine Zahl von 5 bis 20, ist und
die übrigen Variablen die die in Anspruch 1 oder 2 genannten Bedeutungen haben.

4. Cumolhydroperoxidoligomer nach Anspruch 2 oder 3, bei dem das Verhältnis von n zu m in einem Bereich von 0,1 bis 0,9, vorzugsweise 0,5 zu 0,9 liegt.

5. Cumolhydroperoxidoligomer nach einem der Ansprüche 1 bis 4, das eine zahlenmittlere Molmasse von 1000 bis 6 000 g/mol und bevorzugt von 1 000 bis 3 000 g/mol aufweist.

6. Radikalisch polymerisierbare Zusammensetzung, die mindestens ein Cumolhydroperoxidoligomer gemäß einem der Ansprüche 1 bis 5, mindestens ein Thioharnstoffderivat und mindestens ein radikalisch polymerisierbares Monomer enthält.

7. Zusammensetzung nach Anspruch 6, die als Thioharnstoffderivat Acetyl-, Allyl-, Pyridyl-, Phenylthioharnstoff, Hexanoylthioharnstoff oder eine Mischung davon enthält, vorzugsweise Acetylthioharnstoff (ATU).

8. Zusammensetzung nach einem der Ansprüche 6 bis 7, die als radikalisch polymerisierbares Monomer mindestens ein mono- und/oder multifunktionelles (Meth)acrylat, bevorzugt mindestens ein Dimethacrylat oder eine Mischung von Mono- und Dimethacrylaten enthält.

9. Zusammensetzung nach Anspruch 8, die als radikalisch polymerisierbares Monomer
Bisphenol-A-dimethacrylat, Bis-GMA (ein Additionsprodukt aus Methacrylsäure und Bisphenol-A-diglycidylether), ethoxy- oder propoxyliertes Bisphenol-A-Dimethacrylat, 2-[4-(2-Methacryloyloxyethoxyethoxy)phenyl]-2-[4-(2-methacryloyloxyethoxy)phenyl]propan, 2,2-Bis[4-(2-methacryloxypropoxy)-phenyl]propan, UDMA (ein Additionsprodukt aus 2-Hydroxyethylmethacrylat und 2,2,4-Trimethylhexamethylen-1,6-diisocyanat), V-380 (ein Additionsprodukt aus einer Mischung von 0,7 mol 2-Hydroxyethylmethacrylat und 0,3 mol 2-Hydroxypropylmethacrylat mit 1 mol ,α,α,α',α'-Tetramethyl-m-xylylen-diisocyanat), Di-, Tri- oder Tetraethylenglycoldimethacrylat, Trimethylolpropantrimethacrylat, Pentaerythrittetramethacrylat, Glycerindi- und -trimethacrylat, 1,4-Butandioldimethacrylat, 1,10-Decandioldimethacrylat (D₃MA), Bis(methacryloyloxymethyl)tricyclo-[5.2.1.02,6]decan (DCP), ein Polyethylenglycol- oder Polypropylenglycoldimethacrylat, Polyethylenglycol-200- ,Polyethylenglycol-400-dimethacrylat (PEG-200- oder PEG-400-DMA), 1, 1 2-Dodecandioldimethacrylat oder eine Mischung davon und/oder
Benzyl- und Furfurylmethacrylat, 2-Phenoxyethylmethacrylat, 2-(o-Biphenyl-oxy)ethylmethacrylat, 2-Hydroxy-3-phenoxypropyl-methacrylat, Phenethylmethacrylat, 2-[(Benzyloxycarbonyl)-amino]-ethylmethacrylat, 2-[(Benzyl-carbamoyl)oxy]-ethylmethacrylat, 1-Phenoxypropan-2-yl-methacrylat und 2-(p-Cumylphenoxy)-ethylmethacrylat, Tricylodecanmethacrylat, Tricyclodecanmethylmethacrylat und/oder 2-(p-Cumylphenoxy)ethylmethacrylat enthält.

10. Zusammensetzung nach einem der Ansprüche 6 bis 9, die zusätzlich eine Übergangsmetallverbindung enthält, vorzugsweise eine Verbindung eines Übergangsmetalls, das mindestens zwei stabile Oxidationsstufen hat, vorzugsweise eine Verbindung des Kupfers, Eisens, Kobalts, Nickels, Mangans oder eine Mischung davon.

11. Zusammensetzung nach einem der Ansprüche 6 bis 10, die zusätzlich mindestens einen organischen oder anorganischen Füllstoff enthält, vorzugsweise ein Oxid, wie SiO₂, ZrO₂ und TiO₂ oder ein Mischoxid aus SiO₂, ZrO₂, ZnO und/oder TiO₂, einen nanopartikulären oder mikrofeinen Füllstoff, wie pyrogene Kieselsäure oder Fällungskieselsäure, Glaspulver, wie Quarz-, Glaskeramik- oder röntgenopakes Glaspulver, vorzugsweise Barium- oder Strontiumaluminiumsilikatglaspulver, einen röntgenopaken Füllstoff, wie Ytterbiumtrifluorid, Tantal(V)-oxid, Bariumsulfat, ein Mischoxid von SiO₂ mit Ytterbium(III)-oxid oder Tantal(V)-oxid, ein gemahlenes Präpolymerisat oder ein Perlpolymerisat.

12. Zusammensetzung nach einem der Ansprüche 6 bis 11, die
(a) 0,5 bis 15 Gew.-%, bevorzugt 1,0 bis 12,0 Gew.-% mindestens eines Cumolhydroperoxidoligomers der Formel I,
(b) 0,01 bis 5 Gew.-%, bevorzugt 0,05 bis 2,0 Gew.-% mindestens eines Beschleunigers,
(c) 50 bis 95 Gew.-%, bevorzugt 10 bis 95 Gew.-% mindestens eines radikalisch polymerisierbaren Monomers,
(d) 0 bis 80 Gew.-%, bevorzugt 10 bis 80 Gew.-% Füllstoff(e) und
(e) 0,001 bis 5 Gew.-%, bevorzugt 0,01 bis 3 Gew.-% Additiv(e) enthält,
jeweils bezogen auf die Gesamtmasse der Zusammensetzung.

13. Zusammensetzung nach einem der Ansprüche 6 bis 12 zur therapeutischen Behandlung geschädigter Zähne, vorzugsweise als dentaler Zement, Füllungskomposit oder Verblendmaterial.

14. Nicht-therapeutische Verwendung einer Zusammensetzung gemäß einem der Ansprüche 6 bis 14, vorzugsweise zur Herstellung oder Reparatur von Dentalrestaurationen, wie Prothesen, künstlichen Zähnen, Inlays, Onlays, Kronen, Brücken und Totalprothesen.

15. Nicht-therapeutische Verwendung eines Cumolhydroperoxidoligomer gemäß einem der Ansprüche 1 bis 5 als Initiator für die radikalische Polymerisation.

## Claims

1. Cumene hydroperoxide oligomer according to Formula (I): 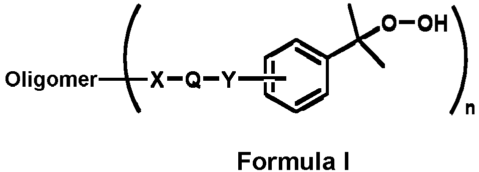
in which the variables have the following meanings:
OLIGOMER is a (meth)acrylate- or styrene-based copolymer chain, which is substituted n times by the group which is in brackets,
Q is a divalent, linear or branched aliphatic C₁-C₁₀ radical, which can be interrupted by 1 to 3 O atoms and can carry 1 to 3 OH or OR² substituents, wherein R² is an aliphatic, linear or branched C₁-C₅ alkyl radical,
X, Y independently of each other are absent, an ether, ester or urethane group, wherein the substitution on the aromatic compound is effected in position 4, and
n is a value from 5 to 20, and
wherein the cumene hydroperoxide oligomer has a number-average molar mass of from 1,000 to 10,000 g/mol, determined by gel permeation chromatography (GPC).

2. Cumene hydroperoxide oligomer according to claim 1, which has the following structure: 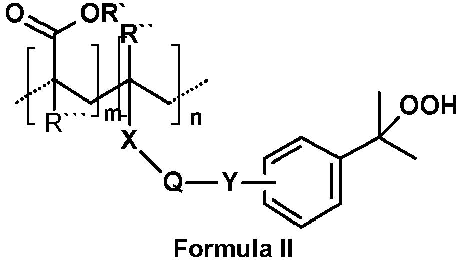 in which
R' is a linear or branched C₁-C₁₀ alkyl radical, which can be substituted by one or more functional groups, in particular -OH, -COOH and/or -Cl, or is unsubstituted, or a cyclic C₄-C₁₀ alkyl radical, a heterocyclic or isocyclic aromatic C₄-C₆ hydrocarbon radical,
R", R‴ independently of each other in each case are H or methyl,
m is a number from 0 to 30, preferably 1 to 20,
n is a number from 5 to 20, and
the remaining variables have the meanings named in claim 1.

3. Cumene hydroperoxide oligomer according to claim 1, which has the following structure: 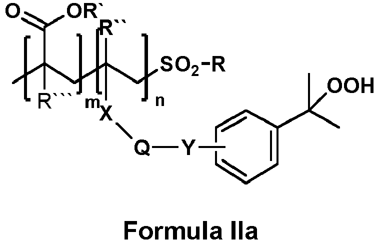 in which
R is a branched or preferably linear C₂-C₁₅ alkyl radical, which can be substituted by one or more functional groups, in particular -Br, -Cl, -OH and/or -COOH, or is preferably unsubstituted,
R' is a linear or branched C₁-C₁₀ alkyl radical, which can be substituted by one or more functional groups, in particular -OH, -COOH and/or -Cl, or is preferably unsubstituted, or a cyclic C₄-C₁₀ alkyl radical, a heterocyclic or isocyclic aromatic C₄-C₆ hydrocarbon radical,
R", R‴ independently of each other in each case are H or methyl,
m is a number from 0 to 30, preferably 1 to 20,
n is a number from 5 to 20, and
the remaining variables have the meanings named in claim 1 or 2.

4. Cumene hydroperoxide oligomer according to claim 2 or 3, in which the ratio of n to m lies in a range of from 0.1 to 0.9, preferably 0.5 to 0.9.

5. Cumene hydroperoxide oligomer according to one of claims 1 to 4, which has a number-average molar mass of from 1,000 to 6,000 g/mol and preferably of from 1,000 to 3,000 g/mol.

6. Radically polymerizable composition, which comprises at least one cumene hydroperoxide oligomer according to one of claims 1 to 5, at least one thiourea derivative and at least one radically polymerizable monomer.

7. Composition according to claim 6, which comprises as thiourea derivative acetyl-, allyl-, pyridyl-, phenylthiourea, hexanoylthiourea or a mixture thereof, preferably acetylthiourea (ATU).

8. Composition according to one of claims 6 to 7, which comprises as radically polymerizable monomer at least one mono- and/or multifunctional (meth)acrylate, preferably at least one dimethacrylate or a mixture of mono- and dimethacrylates.

9. Composition according to claim 8, which comprises as radically polymerizable monomer
bisphenol A dimethacrylate, bis-GMA (an addition product of methacrylic acid and bisphenol A diglycidyl ether), ethoxylated or propoxylated bisphenol A dimethacrylate, 2-[4-(2-methacryloyloxyethoxyethoxy)phenyl]-2-[4-(2-meth-acryloyloxyethoxy)phenyl]propane, 2,2-bis[4-(2-methacryloxypropoxy)phenyl]-propane, UDMA (an addition product of 2-hydroxyethyl methacrylate and 2,2,4-trimethylhexamethylene-1,6-diisocyanate), V-380 (an addition product of a mixture of 0.7 mol 2-hydroxyethyl methacrylate and 0.3 mol 2-hydroxypropyl methacrylate with 1 mol α,α,α',α'-tetramethyl-m-xylylene diisocyanate), di-, tri- or tetraethylene glycol dimethacrylate, trimethylolpropane trimethacrylate, pentaerythritol tetramethacrylate, glycerol di- and trimethacrylate, 1,4-butanediol dimethacrylate, 1,10-decanediol dimethacrylate (D₃MA), bis(methacryloyloxymethyl)tricyclo-[5.2.1.0^{2,6}]decane (DCP), a polyethylene glycol or polypropylene glycol dimethacrylate, polyethylene glycol 200 dimethacrylate, polyethylene glycol 400 dimethacrylate (PEG 200 DMA or PEG 400 DMA), 1,12-dodecanediol dimethacrylate or a mixture thereof and/or
benzyl and furfuryl methacrylate, 2-phenoxyethyl methacrylate, 2-(o-biphenyl-oxy)ethyl methacrylate, 2-hydroxy-3-phenoxypropyl methacrylate, phenethyl methacrylate, 2-[(benzyloxycarbonyl)-amino]-ethyl methacrylate, 2-[(benzylcarbamoyl)oxy]-ethyl methacrylate, 1-phenoxypropan-2-yl methacrylate and 2-(p-cumylphenoxy)-ethyl methacrylate, tricyclodecane methacrylate, tricyclodecane methyl methacrylate and/or 2-(p-cumylphenoxy)ethyl methacrylate.

10. Composition according to one of claims 6 to 9, which additionally comprises a transition metal compound, preferably a compound of a transition metal which has at least two stable oxidation states, preferably a compound of copper, iron, cobalt, nickel, manganese or a mixture thereof.

11. Composition according to one of claims 6 to 10, which additionally comprises at least one organic or inorganic filler, preferably an oxide, such as SiO₂, ZrO₂ and TiO₂ or a mixed oxide of SiO₂, ZrO₂, ZnO and/or TiO₂, a nanoparticulate or microfine filler, such as fumed silica or precipitated silica, glass powder, such as quartz, glass ceramic or radiopaque glass powder, preferably barium or strontium aluminium silicate glass powder, a radiopaque filler, such as ytterbium trifluoride, tantalum(V) oxide, barium sulfate, a mixed oxide of SiO₂ with ytterbium(III) oxide or tantalum(V) oxide, a ground prepolymer or a pearl polymer.

12. Composition according to one of claims 6 to 11, which comprises
(a) 0.5 to 15 wt.-%, preferably 1.0 to 12.0 wt.-%, of at least one cumene hydroperoxide oligomer of Formula I,
(b) 0.01 to 5 wt.-%, preferably 0.05 to 2.0 wt.-%, of at least one accelerator,
(c) 50 to 95 wt.-%, preferably 10 to 95 wt.-%, of at least one radically polymerizable monomer,
(d) 0 to 80 wt.-%, preferably 10 to 80 wt.-%, filler(s) and
(e) 0.001 to 5 wt.-%, preferably 0.01 to 3 wt.-%, additive(s),
in each case relative to the total mass of the composition.

13. Composition according to one of claims 6 to 12 for the therapeutic treatment of damaged teeth, preferably as dental cement, filling composite or veneering material.

14. Non-therapeutic use of a composition according to one of claims 6 to 14, preferably for the production or repair of dental restorations, such as prostheses, artificial teeth, inlays, onlays, crowns, bridges and complete dentures.

15. Non-therapeutic use of a cumene hydroperoxide oligomer according to one of claims 1 to 5 as initiator for the radical polymerization.

## Revendications

1. Oligomère à hydroperoxyde de cumène, de formule (1) : 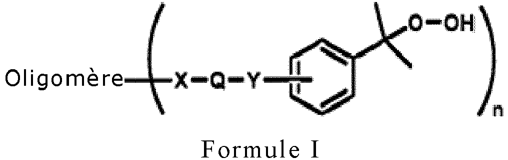
dans laquelle les symboles ont les significations suivantes :
- « Oligomère » représente une chaîne de copolymère, à base d'acrylate ou méthacrylate ou de styrène, qui porte en tant que substituants un nombre n de groupes représentés entre les parenthèses,
- Q représente un reste aliphatique divalent en C₁-C₁₀, linéaire ou ramifié, qui peut être interrompu par 1 à 3 atomes d'oxygène et qui peut porter 1 à 3 substituant(s) symbolisé(s) par OH ou OR², étant entendu que R² représente un groupe aliphatique alkyle en C₁-C₅, linéaire ou ramifié,
- les symboles X et Y, indépendamment l'un de l'autre, ne représentent rien ou représentent un groupe éther, ester ou uréthane, étant entendu que sur le cycle aromatique, ce substituant est placé en position 4,
- et l'indice n vaut de 5 à 20,
lequel oligomère à hydroperoxyde de cumène présente une masse molaire moyenne en nombre, mesurée par GPC (chromatographie par perméation de gel), de 1000 à 10 000 g/mol.

2. Oligomère à hydroperoxyde de cumène, conforme à la revendication 1, qui présente la structure suivante : 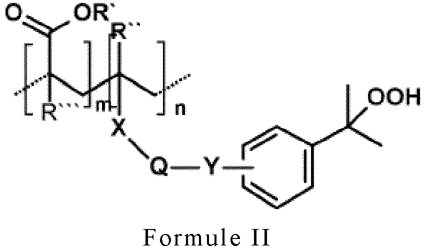 dans laquelle
- R' représente un groupe alkyle en C₁-C₁₀, linéaire ou ramifié, qui peut porter, en tant que substituant(s), un ou plusieurs groupe(s) fonctionnel(s), en particulier groupe(s) hydroxyle, carboxyle et/ou atome(s) de chlore, ou ne porte aucun substituant, ou représente un groupe alkyle cyclique en C₄-C₁₀ ou un reste d'hydrocarbure aromatique hétérocyclique ou isocyclique en C₄-C₆,
- R" et R'" représentent chacun, indépendamment l'un de l'autre, un atome d'hydrogène ou un groupe méthyle,
- l'indice m est un nombre valant de 0 à 30, de préférence de 1 à 20,
- l'indice n est un nombre valant de 5 à 20,
- et les autres symboles ont les significations indiquées dans la revendication 1.

3. Oligomère à hydroperoxyde de cumène, conforme à la revendication 1, qui présente la structure suivante : 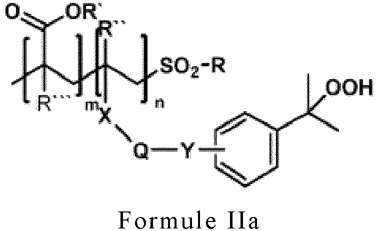 dans laquelle
- R représente un groupe alkyle en C₂-C₁₅, ramifié ou de préférence linéaire, qui peut porter, en tant que substituant(s), un ou plusieurs groupe(s) fonctionnel(s), en particulier atome(s) de brome et/ou de chlore et/ou groupe(s) hydroxyle et/ou carboxyle, mais ne porte de préférence aucun substituant,
- R' représente un groupe alkyle en C₁-C₁₀, linéaire ou ramifié, qui peut porter, en tant que substituant(s), un ou plusieurs groupe(s) fonctionnel(s), en particulier groupe(s) hydroxyle, carboxyle et/ou atome(s) de chlore, mais ne porte de préférence aucun substituant, ou représente un groupe alkyle cyclique en C₄-C₁₀, ou un reste d'hydrocarbure aromatique hétérocyclique ou isocyclique en C₄-C₆,
- R" et R'" représentent chacun, indépendamment l'un de l'autre, un atome d'hydrogène ou un groupe méthyle,
- l'indice m est un nombre valant de 0 à 30, de préférence de 1 à 20,
- l'indice n est un nombre valant de 5 à 20,
- et les autres symboles ont les significations indiquées dans la revendication 1 ou 2.

4. Oligomère à hydroperoxyde de cumène, conforme à la revendication 2 ou 3, dans lequel le rapport de n à m se situe dans l'intervalle allant de 0,1 à 0,9 et de préférence, de 0,5 à 0,9.

5. Oligomère à hydroperoxyde de cumène, conforme à l'une des revendications 1 à 4, qui présente une masse molaire moyenne en nombre de 1000 à 6000 g/mol, et de préférence de 1000 à 3000 g/mol.

6. Composition polymérisable par voie radicalaire, qui contient au moins un oligomère à hydroperoxyde de cumène conforme à l'une des revendications 1 à 5, au moins un dérivé de thiourée, et au moins un monomère polymérisable par voie radicalaire.

7. Composition conforme à la revendication 6, qui contient, en tant que dérivé de thiourée, de l'acétyl-thiourée, de l'allyl-thiourée, de la pyridyl-thiourée, de la phényl-thiourée, de l'hexanoyl-thiourée, ou un mélange de ces composés, et de préférence de l'acétyl-thiourée (ATU).

8. Composition conforme à l'une des revendications 6 et 7, qui contient, en tant que monomère polymérisable par voie radicalaire, au moins un acrylate ou méthacrylate monofonctionnel et/ou polyfonctionnel, et de préférence, au moins un diméthacrylate ou un mélange de monométhacrylate et de diméthacrylate.

9. Composition conforme à la revendication 8, qui contient, en tant que monomère polymérisable par voie radicalaire, du diméthacrylate de bisphénol A, du bis-GMA (produit d'addition obtenu à partir d'acide méthacrylique et d'éther diglycidylique de bisphénol A),
du diméthacrylate de bisphénol A éthoxylé ou propoxylé, du 2-[4-(2-méthacryloyloxy-éthoxy-éthoxy)-phényl]-2-[4-(2-méthacryloyloxy-éthoxy)-phényl]-propane, du 2,2-bis[4-(2-méthacryloxy-propoxy)-phényl]-propane, de l'UDMA (produit d'addition obtenu à partir de méthacrylate de 2-hydroxy-éthyle et de 2,2,4-triméthyl-hexaméthy-lène-1,6-diisocyanate), du V380 (produit d'addition obtenu à partir d'un mélange de 0,7 mole de méthacrylate de 2-hydroxy-éthyle et de 0,3 mole de méthacrylate de 2-hydroxy-propyle et 1 mole d'α,α,α,'α'-tétraméthyl-méta-xylylène-diisocyanate, du diméthacrylate de diéthy-lène-glycol, triéthylène-glycol ou tétraéthylène-glycol, du triméthacrylate de triméthylol-propane, du tétraméthacrylate de pentaérythritol, du diméthacrylate ou triméthacrylate de glycérol, du diméthacrylate de butane-1,4-diol, du diméthacrylate de décane-1,10-diol (D₃MA), du bis(méthacryloyloxy-méthyl)-tricyclo[5.2.1.0(2,6)]décane (DCP), un diméthacrylate de polyéthylène-glycol ou de polypropylène-glycol, du diméthacrylate de polyéthylène-glycol-200 ou de polyéthylène-glycol-400 (PEG-200-DMA ou PEG-400-DMA), du diméthacrylate de dodécane-1,12-diol ou un mélange de ces composés, et/ou du méthacrylate de benzyle et du méthacrylate de furfuryle, du méthacrylate de 2-phénoxy-éthyle, du méthacrylate de 2-(ortho-biphényl-oxy)-éthyle, du méthacrylate de 2-hydroxy-3-phénoxy-propyle, du méthacrylate de phénéthyle, du méthacrylate de 2-[(benzyloxy-carbonyl)-amino]-éthyle, du méthacrylate de 2-[(benzyl-carbamyl)-oxy]-éthyle, du méthacrylate de 1-phénoxy-prop-2-yle, et du méthacrylate de 2-(para-cumylphénoxy)-éthyle, du méthacrylate de tricyclodécyle, du méthacrylate de tricyclodécyl-méthyle, et/ou du méthacrylate de 2-(para-cumylphénoxy)-éthyle.

10. Composition conforme à l'une des revendications 6 à 9, qui contient en outre un composé de métal de transition, et de préférence, un composé d'un métal de transition qui possède au moins deux états stables d'oxydation, et mieux encore, un composé du cuivre, du fer, du cobalt, du nickel ou du manganèse, ou un mélange de tels composés.

11. Composition conforme à l'une des revendications 6 à 10, qui contient en outre au moins une charge organique ou inorganique, de préférence un oxyde, tels SiO₂, ZrO₂ et TiO₂, ou un oxyde mixte à base de SiO₂, ZrO₂, ZnO et/ou TiO₂, une charge micro-fine ou en nano-particules, comme de la silice pyrogénée ou de la silice précipitée, de la poudre de verre, comme de la poudre de quartz, de la poudre de vitrocéramique ou de la poudre de verre opaque aux rayons X, de préférence de la poudre de verre aluminosilicate de baryum ou de strontium, une charge opaque aux rayons X, tels du trifluorure d'ytterbium, de l'oxyde de tantale-(V), du sulfate de baryum, et un oxyde mixte à base de SiO₂ et d'oxyde d'ytterbium-(III) ou d'oxyde de tantale-(V), un produit de prépolymérisation broyé ou un produit de polymérisation en perles.

12. Composition conforme à l'une des revendications 6 à 11, qui contient :
a) de 0,5 à 15 % en poids, et de préférence de 1,0 à 12,0 % en poids, d'au moins un oligomère à hydroperoxyde de cumène de formule (I),
b) de 0,01 à 5 % en poids, et de préférence de 0,05 à 2,0 % en poids, d'au moins un accélérateur,
c) de 50 à 95 % en poids, et de préférence de 10 à 95 % en poids, d'au moins un monomère polymérisable par voie radicalaire,
d) de 0 à 80 % en poids, et de préférence de 10 à 80 % en poids, de charge(s),
e) et de 0,001 à 5 % en poids, et de préférence de 0,01 à 3 % en poids, d'adjuvant(s),
par rapport, dans chaque cas, au poids total de la composition.

13. Composition, conforme à l'une des revendications 6 à 12, pour traitement thérapeutique de dents abimées, de préférence en tant que ciment dentaire, composite d'obturation ou matériau de placage.

14. Utilisation non-thérapeutique d'une composition conforme à l'une des revendications 6 à 14, de préférence pour la fabrication ou la réparation de pièces de restauration dentaire, telles que prothèses, dents artificielles, incrustations inlays ou onlays, couronnes, bridges et prothèses totales.

15. Utilisation non-thérapeutique d'un oligomère à hydroperoxyde de cumène, conforme à l'une des revendications 1 à 5, en tant qu'amorceur pour polymérisation par voie radicalaire.
